(19) Europäisches Patentamt
European Patent Office
Office européen des brevets

(11) **EP 3 879 537 A1**

(12) **DEMANDE DE BREVET EUROPEEN**

(43) Date de publication:
**15.09.2021 Bulletin 2021/37**

(51) Int Cl.:
***G16B 30/00*** (2019.01)   ***G16B 20/00*** (2019.01)
***G16B 40/20*** (2019.01)

(21) Numéro de dépôt: **20162655.3**

(22) Date de dépôt: **12.03.2020**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(71) Demandeur: **bioMérieux**
**69280 Marcy l'Etoile (FR)**

(72) Inventeurs:
• **JAILLARD DANCETTE, Magali**
**69280 Marcy l'Etoile (FR)**
• **MAHE, Pierre**
**35250 Lans en Vercors (FR)**

(74) Mandataire: **Le Mauff, Frédéric Francis Joseph**
**bioMérieux**
**376, chemin de l'Orme**
**69280 Marcy-l'Étoile (FR)**

(54) **TECHNOLOGIE MOLÉCULAIRE DE PRÉDICTION D'UN CARACTÈRE PHÉNOTYPIQUE D'UNE BACTÉRIE À PARTIR DE SON GÉNOME**

(57) Un procédé de détermination d'une caractéristique phénotypique d'une souche bactérienne, comprend le séquençage (400) partiel ou total du génome de ladite souche, et l'application (402,404), au génome séquencé, d'un modèle prédéterminé de prédiction de ladite caractéristique, ledit modèle ayant pour variables des groupes de séquences génomiques.

Selon l'invention, lesdits groupes sont sélectionnés (304, 312) de sorte :
- qu'un taux de co-occurrence, dans le génome de l'espèce bactérienne, des séquences génomiques constitutives desdits groupes est supérieur à un seuil prédéterminé (304); et
- que les groupes sont des regroupements desdites séquences génomiques en fonction de leurs taux de co-occurrence dans le génome de l'espèce bactérienne (312).

Figure 2A

EP 3 879 537 A1

**Description**

**DOMAINE DE L'INVENTION**

La présente invention a trait au domaine technique de la biologie moléculaire appliquée à la génomique bactérienne, et en particulier au domaine de la prédiction de traits phénotypiques de bactéries à partir de leurs génomes. L'invention trouve particulièrement application dans la prédiction de la susceptibilité aux antibiotiques et de la virulence de bactéries présentes dans un échantillon biologique.

**ETAT DE LA TECHNIQUE**

A. TECHNOLOGIE MOLECULAIRE DE PREDICTION PHENOTYPIQUE

La susceptibilité d'une souche bactérienne à un antibiotique, c'est-à-dire son caractère sensible ou résistant dans le cadre d'un traitement à base de l'antibiotique administré à un humain ou un animal, n'est pas directement observable par un être humain. En effet, une observation directe de la souche, même au travers de microscopes, ne permet pas de déterminer son comportement face à l'antibiotique. Le diagnostic *in vitro* en matière bactérienne consiste, par nature, à rendre ce caractère phénotypique observable et donc in fine exploitable pour un clinicien. Au cours du XXe siècle les technologies de diagnostic *in vitro* ont essentiellement combiné des techniques de préparation d'échantillon à base de culture, notamment pour rendre visibles et manipulables les souches bactériennes présentes dans les échantillons, et des techniques de mesures optiques du comportement des souches en présence d'un antibiotique. Par exemple, un flux de travail classique d'un laboratoire microbiologique consiste dans un premier temps à étaler sur un milieu de culture un échantillon prélevé sur un patient suspecté d'infection bactérienne afin de faire apparaître, après incubation, des colonies bactériennes visibles par un opérateur humain ou un automate. Dans un second temps, la taille des colonies étant suffisante, un technicien ou un automate prélève une colonie, la mélange à un antibiotique à différentes concentrations et introduit les mélanges dans un dispositif qui mesure la densité optique de chaque mélange et en déduit la susceptibilité à l'antibiotique. La densité optique signant la prolifération bactérienne, elle caractérise alors sans ambiguïté la sensibilité ou la résistance de la bactérie : si la densité augmente, cela signifie qu'elle prolifère malgré la présence de l'antibiotique, et donc qu'elle est résistante à ce dernier à la concentration antibiotique considérée.

Aujourd'hui la combinaison des technologies de préparation d'échantillon et des technologies de mesures optiques à base de densité optique montre d'importantes limitations face à une évolution rapide à l'échelle mondiale dans le règne des procaryotes, à savoir l'acquisition de multi-résistances aux antibiotiques, multi-résistances dont on estime qu'elle fera, en 2050, plus de morts que le cancer. Tout d'abord, ces technologies ne sont pas agnostiques concernant les bactéries. En effet, en fonction du milieu de culture choisi, certaines souches vont pousser et d'autres non, de sorte que ces technologies ne permettent pas de caractériser la susceptibilité aux antibiotiques de toutes les espèces bactériennes. Ensuite, ces techniques sont extrêmement lentes car elles se basent sur une culture bactérienne qui prend beaucoup de temps. C'est ainsi qu'obtenir un antibiogramme d'une bactérie prend au moins 30h heures à partir du prélèvement de l'échantillon. Ce délai ne permet pas un traitement efficace des patients qui se voient administrer en première intention, et de manière systématique, un cocktail antibiotique à large spectre. Outre les conséquences pour le patient, cette administration inappropriée et massive d'antibiotique renforce la pression de sélection des bactéries multi-résistantes et contribue donc à leur expansion. A ce jour donc, on estime que les technologies classiques de diagnostic *in vitro* sont de moins en moins adaptées au traitement des patients et sont, dans une certaine mesure, une des raisons de l'apparition des multi-résistances.

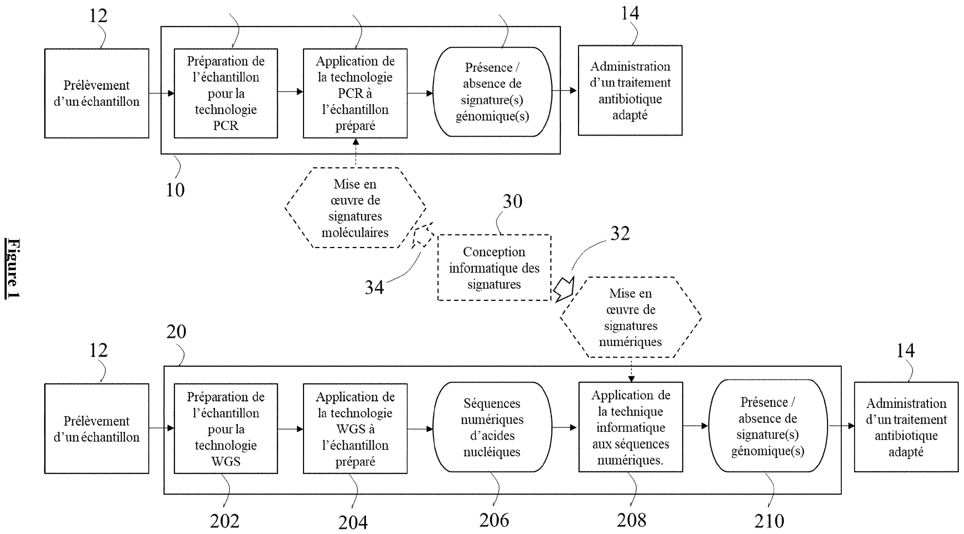

La maturation des technologies de biologies moléculaire, en particulier les technologies de caractérisations de l'ADN et/ou l'ARN bactérien, telle que la réaction en chaîne par polymérase (ou PCR), les puces à ADN ou le séquençage, réalise un changement de paradigme dans l'analyse de la résistance aux antibiotiques dans les laboratoires. Tout d'abord, elles sont plus agnostiques vis-à-vis des espèces bactériennes. Par exemple, la technologie métagénomique permet de traiter l'ADN bactérien dans un échantillon biologique quelles que soient les espèces bactériennes en présence. Ensuite, elles ont pour objectif de rendre un résultat en quelques heures, certaines comme la PCR permettant même un résultat en moins de 20 minutes. En contrepartie, les techniques moléculaires de caractérisation de la susceptibilité aux antibiotiques reposent sur des signatures génomiques (absence/présence de gènes, de mutations génétiques, modèles de prédiction, etc.) caractérisant ladite susceptibilité. La **figure 1** illustre de manière simplifiée, et non limitative, deux technologies de caractérisation de l'ADN bactérien, à savoir une technologie PCR **10** et une technologie de séquençage de génome complet **20** (ou séquençage WGS pour « whole genome sequencing ») appliquée dans le cadre de flux de travail microbiologique pour le traitement d'un patient suspecté d'une infection bactérienne. Les deux flux débutent par le prélèvement **12** d'un échantillon biologique sur le patient, suivi par l'application de la technologie PCR **10** ou de la technologie WGS **20,** rendant chacune un résultat **106, 210** de signatures génomiques caractérisant la susceptibilité à un ou plusieurs antibiotiques, résultat sur la base duquel un traitement antibiotique est choisi et administré au patient par un clinicien en **14.** De manière basique, chacune des technologies moléculaires nécessite la préparation **102, 202** de l'échantillon prélevé préalablement à l'application de la PCR elle-même **104,** par exemple une PCR de type « nested » mise en œuvre par une plateforme Filmarray de la société Biofire, ou à l'application du séquençage **204,** par exemple un séquençage de type SBS mise en œuvre par une plateforme MiSeq de la société Illumina.

Une des principales différences entre ces deux technologiques moléculaires est la nature des signatures génomiques. Dans le cadre de la PCR, la signature génomique est moléculaire, et donc tangible : elle est traduite en amorces introduites dans un mélange réactionnel, amorces qui ciblent de manière spécifique des séquences d'ADN bactérien introduite dans le mélange, et sa détection est réalisée généralement par la mesure d'un signal optique. A contrario, dans le cadre du WGS, les signatures génomiques sont numériques puisque le séquençage produit un génome numérique et que le traitement dudit génome est informatique. Si la technologie WGS permet, a minima, la mise en œuvre des signatures génomiques de la PCR, elle autorise surtout une exploitation complexe du génome numérique et l'emploi de modèles de prédiction de la susceptibilité aux antibiotiques impossible à mettre en œuvre par les technologies PCR. Ainsi, la technologie WGS s'appuie avantageusement sur une conception informatique des signatures génomiques **30,** conception qui exploite avantageusement de larges bases de connaissances génomiques et phénotypiques à l'aide d'outils analytique complexe, par exemple des technologies d'apprentissage automatisé (« machine learning ») telles que les régressions logistiques sous contrainte de parcimonie.

Ceci étant posé, toutes les techniques moléculaires reposent sur un même fondamental technique, à savoir la mesure d'une information génomique des souches bactériennes et le traitement de ladite information afin d'en extraire une information sur le comportement des souches en présence d'un antibiotique. De plus, si ces technologies informatiques, et plus particulièrement celles du séquençage, sont de nature différente de celles associées à l'analyse de densité optique des technologies microbiologiques plus classiques, elles ne changent pas la nature technique du diagnostic *in vitro* qu'elles mettent en œuvre. Concernant le diagnostic infectieux par exemple, il s'agit toujours d'appliquer des technologies de traitement d'un échantillon biologique afin de déterminer si un patient est atteint d'une infection bactérienne et de connaitre le comportement de la bactérie infectieuse en présence d'un antibiotique afin d'administrer un traitement antibiotique approprié.

## B. INTERPRETABILITE DES TECHNOLOGIES MOLECULAIRES DE PREDICTION PHENOTYPIQUE

En se focalisant plus particulièrement sur les signatures génomiques, les première approches consistaient à identifier dans le génome bactérien des marqueurs de résistance aux antibiotiques identifiés au préalable, approches qualifiées « d'association directe». Si ces approches sont efficaces lorsque les mécanismes génétiques entrainant la résistance sont bien connus et simples, comme cela est le cas de la plupart des mécanismes de résistances chez des espèces comme par exemple *Mycobacterium tuberculosis,* elles souffrent d'importantes limitations : connaissance incomplète des mécanismes de résistances chez beaucoup d'espèces et d'antibiotiques, se traduisant par exemple par des bases de données incomplètes, difficulté à tenir compte de différences dans les pouvoirs prédictifs des marqueurs et de l'aspect multifactoriel de la susceptibilité aux antibiotiques (e.g. épistasie, combinaison de multiples mutations,...), etc. Face à ces difficultés, le déterminisme génétique de la susceptibilité aux antibiotiques est appréhendé plus efficacement par de nouvelles approches à base de technologies informatiques avancée, et en particulier par des technologies d'apprentissage automatique supervisé dont l'architecture d'apprentissage et d'application peut être résumé de la manière suivante :

A. pour un ensemble de souches bactériennes d'apprentissage :

a. chaque souche est séquencée et phénotypiquement caractérisée (e.g. mesure de sa concentration minimale inhibitrice et/ou mesure de sa susceptibilité - résistance, intermédiaire ou sensible- pour un ou plusieurs antibiotiques).
b. un modèle informatique de prédiction de la susceptibilité à l'antibiotique est appris en fonction des génomes et des données phénotypiques.

B. pour une nouvelle souche dont on cherche à connaître la susceptibilité à un antibiotique de l'étape (A. a) :

a. la souche est séquencée ;
b. la modèle de prédiction informatique est appliqué à son génome numérique afin de déterminer sa susceptibilité.

La description générique ci-dessus suppose en premier lieu de définir les variables de l'apprentissage automatique qui décrivent le génome bactérien. Il existe de nombreuses manières de décrire ce dernier, l'une d'entre elle étant la description en « k-mers », à savoir la liste des séquences d'acides nucléiques de longueur *k* (i.e. nombre de bases) constitutives du génome. Comme décrit dans la thèse de M. Jaillard Dancette, « Vers une cartographie des polymormismes liés à la résistance aux antimicrobiens », 2018, cette description est particulièrement bien adaptée aux génomes bactériens qui sont haploïdes et très plastiques comparativement aux génomes des eucaryotes. Dit autrement, cette description permet de décrire efficacement, chez les bactéries, la diversité des mécanismes génétiques à l'origine de leur susceptibilité aux antibiotiques.

Cette description présente cependant un certain nombre de désavantages pouvant impacter de manière négative les technologies d'apprentissage automatique, notamment :

a. les k-mers sont fortement redondants : ceux couvrant une région génomique conservée peuvent être co-occurrents, i.e. être présents ou absents de manière systématique dans un ensemble de génomes, et sont donc statistiquement équivalents.

b. certains k-mers ne sont pas spécifiques d'une région génomique de sorte qu'ils sont difficiles à annoter, c'est-à-dire à caractériser structurellement ou fonctionnellement (gène, mutation, etc..).

c. l'association génome-susceptibilité est un problème de très grande dimension, le nombre de k-mers par génome étant supérieur à la centaine de milliers, voire au million, de sorte que la redondance et la non spécificité conduisent à des variables fortement corrélées pour les outils d'apprentissage.

Pour les domaines à fort risque, en particulier celui de la santé humaine, il est important de réduire la dimensionnalité des variables pour augmenter l'interprétabilité des modèles de prédiction. En effet, les outils d'apprentissage automatique sont sensibles aux biais dans les données d'apprentissage, par exemple le manque de diversité génomique, et aux biais associés à une formulation incomplète de la susceptibilité en fonction du génome bactérien, par exemple la non prise en compte des fortes corrélations entre différentes régions génomiques. En réduisant la dimensionnalité, les modèles de prédiction deviennent plus simples à interpréter à la fois pour l'expert de l'outil d'apprentissage et pour l'expert de la génomique bactérienne, ce qui permet de détecter les biais et ainsi construire des données d'apprentissage appropriées ou reformuler le problème que l'outil d'apprentissage a pour objet de résoudre. De la même manière, étant mieux interprétables, les modèles de prédiction sont plus simples à valider pour leur utilisation dans un domaine à fort risque si aucun biais n'est apparent après leur analyse.

Parmi les outils permettant une forte réduction de la dimensionnalité, les outils d'apprentissage automatiques parcimonieux, comme par exemple les régressions de type lasso pénalisées ou les outils à base d'arbres décisionnels, permettent d'obtenir un nombre de k-mers prédictifs, c'est-à-dire retenus dans le modèle de prédiction, de l'ordre du millier, voire de l'ordre de la centaine. Ces outils sont cependant instables dans un environnement de haute dimensionnalité dans lequel les variables sont fortement corrélées. Ainsi, ils peuvent sélectionner des variables prédictives formant ensemble un motif génomique qui n'a pas nécessairement de réalité biologique, de sorte que les modèles de prédiction restent difficilement interprétables. Certaines techniques permettent de tenir compte des fortes corrélations entre les variables, notamment les régressions à base de pénalité « elastic-net » qui combinent les pénalités de type L1 avec des pénalités de type L2, ce qui mène à sélectionner des groupes de variables prédictives corrélées. On note cependant que ce regroupement reste principalement algorithmique et que les groupes de variables retenu sont encore difficilement interprétables biologiquement.

D'autres outils, comme par exemple les outils de type « group-lasso », permettent le regroupement *a priori* de variable descriptives en motifs génomiques. Dans ce cadre, l'ensemble des variables d'un motif sont prédictives ou ne le sont pas en fonction de la sélection ou de la non sélection du motif par la stratégie group-lasso. Toutefois, la description en k-mers étant difficilement interprétable pour les raisons évoquées ci-dessus, la définition *a priori* en motifs explicatifs est difficile. En particulier, cela suppose que des phénomènes de forte corrélation dans un espace de haute dimension soient bien compris et formalisés par les experts de la génomique bactérienne puisqu'un manque de connaissance, ou une connaissance imparfaite de ces phénomènes, se traduit en biais dans l'algorithme d'apprentissage automatique.

C. APPLICATION DES TECHNOLOGIES MOLECULAIRES DE PREDICTION FACE A LA VARIABILITE BIOLOGIQUE

Parallèlement à la problématique exposée ci-dessus, lorsqu'une prédiction est basée sur des groupes de séquences génomiques, la question se pose de savoir si un groupe est présent dans le génome d'une souche si toutes les séquences qui le constituent sont toutes présentes dans le génomes et à l'identique. Si un tel critère est appliqué, cela suppose que les données d'apprentissage soient complètes pour englober toute la variabilité génomique de l'espèce bactérienne. Outre qu'il est difficile, à un moment donnée, de juger de la complétude des données d'apprentissage, ces dernières deviennent très souvent incomplètes au fil du temps chez certaines espèces bactériennes en raison de la plasticité très importante de leur génome. Aussi, l'application d'un critère trop strict mène à un taux de faux positif ou de faux négatif élevé.

Les problématiques venant d'être décrites en relation avec la prédiction génomique de la susceptibilité à un ou plusieurs antibiotiques d'une souche bactérienne se posent dans les mêmes termes pour toute détermination génomique d'une caractéristique phénotypique de la souche, par exemple sa virulence ou son ribotype.

**EXPOSE DE L'INVENTION**

Le but de la présente invention est de proposer une prédiction génomique d'une caractéristique phénotypique, en particulier de la susceptibilité à un antibiotique, qui soit plus représentatif de la réalité biologique de ladite caractéristique de l'espèce bactérienne à laquelle appartient la souche tout en conservant les performances de la prédiction.

A cet effet l'invention a pour objet un procédé de détermination d'une caractéristique phénotypique d'une souche bactérienne, comprenant :

- le séquençage partiel ou total du génome de ladite souche;
- l'application, au génome séquencé, d'un modèle prédéterminé de prédiction de ladite caractéristique, ledit modèle ayant pour variables des groupes de séquences génomiques.

Selon l'invention, lesdits groupes sont sélectionnés de sorte :

- qu'un taux de co-occurrence, dans les génomes de l'espèce bactérienne auquel appartient ladite souche, des séquences génomiques constitutives desdits groupes est supérieur à un seuil prédéterminé ; et
- que les groupes sont des regroupements desdites séquences génomiques en fonction de leurs taux de co-occurrence dans les génomes de l'espèce bactérienne.

En d'autres termes, l'invention contraint l'apprentissage automatique à considérer les séquences génomiques de poids équivalents dans la prédiction de la caractéristique phénotypique comme des groupes plutôt que des variables indépendantes. Les inventeurs ont ainsi noté que la prédiction ne voit pas ses performances dégradées alors que, dans le mêmes temps, le ou les groupes appris qui ont le plus de poids prédictifs correspondent à une réalité biologique, par exemple correspondent à des gènes entiers ou des groupes de mutations spécifiques. Cette approche a notamment été validée sur des espèces bactériennes dont l'origine génomique de la susceptibilité est bien documentée.

Dans ce qui suit, une application à la susceptibilité à un antibiotique est décrite, étant entendu que l'application vaut également pour toute caractéristique phénotypique.

Selon un mode de réalisation de l'invention, dans lequel un premier sous ensemble des séquences génomiques est un ensemble de variables non groupées d'un premier modèle de prédiction de la susceptibilité. En d'autres termes, ce filtrage permet d'éliminer d'emblée les séquences n'ayant pas de poids prédictif en utilisant de manière avantageuse des outils d'apprentissage non contraints pour le regroupement, et donc a priori les plus performants.

En particulier, le premier modèle de prédiction est un modèle appris sur une base de données de génomes et de susceptibilités de souches bactériennes appartenant à l'espèce bactérienne, l'apprentissage appartenant à la classe des apprentissage parcimonieux, avantageusement une régression logistique, notamment de type LASSO. Par « apprentissage parcimonieux », on entend un apprentissage qui définit un modèle de prédiction dont les variables forment un sous ensemble des variables descriptives d'un problème, visant à minimiser la taille de ce sous-ensemble sans impacter la performance de prédiction du modèle.

En d'autres termes, les approches parcimonieuses permettent de sélectionner un premier jeu de séquences génomiques très prédictives auquel s'ajouteront d'autres séquences qui ont des schémas de co-occurrence très proches. Plus particulièrement, une approche parcimonieuse permet d'identifier des régions du génome impliquées dans la susceptibilité à l'antibiotique. De manière avantageuse, un second sous ensemble des séquences génomiques est un ensemble de séquences génomiques ayant un taux de co-occurrence avec le premier ensemble supérieur au seuil prédéterminé. En d'autres termes, si les approches parcimonieuses permettent une première identification des régions génomiques d'intérêt, elles échouent généralement, en raison des corrélations entre variables, à décrire de manière exhaustive lesdites régions. En étendant le premier ensemble de séquences obtenus par l'apprentissage parcimonieux par un second ensemble de séquences co-occurrentes, un ensemble de séquence permettant une description exhaustive desdites régions est généré.

Selon un mode de réalisation, les séquences génomiques correspondent à des nœuds d'un graphe compacté obtenu à partir de séquences génomiques de longueur constante, de longueur k en nombre de bases, ou « k-mers », et occurrentes dans des génomes de souches appartenant à l'espèce bactérienne. En particulier, ledit graphe compacté est calculé en a) calculant un graphe de De Buijn d'alphabet A, T, G, C à partir desdites séquences génomiques de longueur constante, b) en compactant des chemins linéaires du graphes de De Bruijn de manière à obtenir ledit graphe compacté. De cette manière, il est obtenu un ensemble de séquences génomiques uniques, la redondance existant dans les séquences descriptives de longueur constante, à savoir les k-mers, étant éliminée.

Selon un mode de réalisation, le regroupement des séquences génomiques comporte :

- l'obtention de premiers groupes de séquences génomiques par l'application d'un regroupement des séquences ayant une co-occurente parfaite dans des génomes de souches appartenant à l'espèces bactérienne ;

- l'obtention des groupes du modèle de prédiction par l'application d'un second regroupement desdits premiers groupes basé sur les taux de co-occurrence desdits premiers groupes dans lesdits génomes.

De cette manière, les séquences qui sont systématiquement présentes en même temps dans chacun des génomes des souches de l'espèce bactérienne ayant servi à construire le graphe compacté, ou absentes en même temps, ou certaines présentes et d'autres absentes en même temps, sont regroupées en motifs génomiques uniques, diminuant le nombre de variables, et également une redondance cachée.

Selon un mode de réalisation, la valeur d'un groupe de séquences génomiques pour l'application du modèle de prédiction appliqué à la souche bactérienne est égale au pourcentage desdites premiers groupes dudit groupe présents dans le génome de la souche bactérienne (328).

Selon un mode de réalisation, la valeur d'un groupe de séquences génomiques pour l'application du modèle de prédiction appliqué à la souche bactérienne est calculée en :

- calculant, pour chaque premier groupe dudit groupe, le pourcentage de séquences génomiques dudit premier groupe présentes dans le génomes de la souche bactérienne ; et
- en calculant la valeur comme étant égale à la moyenne des pourcentages des premiers groupes dudit groupe.

Selon un mode de réalisation, le regroupement des séquences génomiques comporte a) le calcul d'un dendrogramme en fonction des taux de co-occurence des séquences génomiques dans des génomes de souches appartenant à l'espèce bactérienne, et b) la partition du dendrogramme à une hauteur prédéterminée de manière à obtenir les groupes de séquences génomiques. En particulier, le dendrogramme est calculé sur les premiers groupes de séquences génomiques. Cette approche hiérarchique basée sur l'occurrence permet de régler de manière compréhensible le nombre de groupes de séquences. En jouant sur la hauteur de partition, il est possible d'optimiser la valeur biologique des groupes sélectionnés.

Selon un mode de réalisation, le modèle de prédiction est appris sur une base de données de génomes et de susceptibilités de souches bactériennes appartenant à l'espèce bactérienne, l'algorithme d'apprentissage appartenant à la classe des apprentissages parcimonieux, en particulier à base de régression logistique. En particulier, l'apprentissage est réalisé par une régression logistique, notamment de type LASSO. Par l'approche parcimonieuse, les groupes les plus prédictifs sont sélectionnés, ces groupes constituant des signatures génomiques de la susceptibilité.

De manière avantageuse, la valeur d'un groupe de séquences génomiques pour l'apprentissage parcimonieux est calculé en

- pour chaque séquence génomique appartenant audit groupe, en calculant un vecteur d'occurrences de ladite séquence dans les génomes de la base de données ;
- en calculant le vecteur moyen des vecteurs d'occurrences, ledit vecteur moyen constituant la valeur dudit groupe.

Selon un mode de réalisation, la valeur d'un groupe de séquences génomiques pour le modèle de prédiction appliqué à la souche bactérienne est égale au pourcentage de séquences génomiques dudit groupe présentes dans le génome de la souche bactérienne. En particulier, une séquence génomique est présente dans le génome de la souche bactérienne lorsqu'un pourcentage de séquences génomiques de longueur constante constitutives de ladite séquence est supérieur à un seuil prédéterminé.

L'invention a également pour objet un procédé d'identification de signatures génomiques prédictives de la susceptibilité à un antibiotique d'une espèce bactérienne, le procédé comprenant :

- la constitution d'une base de données de génomes et de susceptibilité d'une pluralité de souches bactériennes appartenant à l'espèce bactérienne ;
- le calcul d'un ensemble de séquences génomiques descriptives des génomes de la base de données, et pour chacune desdites séquences le calcul d'un vecteur d'occurrence de ladite séquence dans les génomes de la base de données ;
- la sélection, dans ledit ensemble de séquences génomiques, des séquences ayant un taux de co-occurrence supérieur à un seuil prédéterminé, ledit taux étant calculé en fonction des vecteurs d'occurrence ;
- le regroupement des séquences génomiques sélectionnées en fonction de leurs taux de co-occurrence, de manière à obtenir des groupes de séquences génomiques ;
- l'apprentissage d'un modèle de prédiction de la susceptibilité de l'espèce bactérienne à l'antibiotique ayant pour variables les groupes de séquences génomiques ;
- l'identification de la signature comme étant égale à l'union de groupes de séquences génomiques.

L'invention a également pour objet une utilisation d'une signature génomique identifiée selon un procédé d'identification de signatures génomiques du type précité, dans un test moléculaire de prédiction d'une caractéristique phénotypique, en particulier de la susceptibilité à un antibiotique, en particulier un test de type réaction en chaîne par polymérase, ou un test à base de puce à ADN, ledit test ciblant au moins partiellement la signature génomique.

L'invention a également pour un produit programme d'ordinateur mémorisant des instructions exécutables par un ordinateur pour l'application d'un modèle de prédiction d'une caractéristique phénotypique, en particulier de la susceptibilité à un antibiotique, d'une souche bactérienne du type précité.

L'invention a également pour objet un système de détermination de la susceptibilité à un antibiotique d'une souche bactérienne, comprenant :

- un plateforme de séquençage pour le séquençage partiel ou total du génome de ladite souche;
- une unité informatique configurée pour l'application, au génome séquencé, d'un modèle prédéterminé de prédiction d'une caractéristique phénotypique du type précité.

L'invention a également pour objet un produit programme d'ordinateur mémorisant des instructions exécutables par un ordinateur pour la mise en œuvre d'un procédé d'identification de signatures génomiques prédictives d'une caractéristique phénotypique, en particulier de la susceptibilité à un antibiotique, d'une souche bactérienne du type précité.

**BREVE DESCRIPTION DES FIGURES**

L'invention sera mieux comprise à la lecture de la description qui suit, donnée uniquement à titre d'exemple, et réalisée en relation avec les dessins annexés, dans lesquels des références identiques désignent des éléments identiques ou analogues, et dans lesquels :

- la **figure 1** illustre deux technologies moléculaires de l'état de la technique pour la prédiction de la susceptibilité à un antibiotique d'une souche bactérienne ;
- les **figures 2A et 2B** sont des organigrammes d'une phase d'apprentissage et d'une phase de prédiction selon l'invention ;
- la **figure 3** illustre la génération de motif MAF selon l'invention ;
- la **figure 4** illustre un clustering utilisé dans le procédé selon l'invention ;
- la **figure 5** est une courbe ROC illustrant la sélection d'un seuil de prédiction selon l'invention ;
- la **figure 6** illustre, pour l'espèce bactérienne *Klebsiella pneumoniae* et l'antibiotique méropénème, les coefficients d'un modèle de prédiction obtenu selon une technique lasso et selon le procédé de l'invention, nommé « cluster-lasso », ainsi que la localisation des variables prédictives de deux modèles lasso et cluster-lasso dans le sous-graphes du graphe compacté annoté comme étant gène *blaKPC* ;
- la **figure 7** illustre, pour l'espèce bactérienne *K. pneumoniae* et l'antibiotique céfoxitine, le ou les sous-graphes du graphe compacté, impliquant les motifs MAF étendus les plus prédictifs du modèle lasso (partie de gauche), et le ou les sous-graphes du graphe compacté impliquant les clusters les plus prédictifs du modèle cluster-lasso (partie de droite) ;
- la **figure 8** illustre, pour l'espèce *Salmonella* et pour l'antibiotique tétracycline, les valeurs absolues des coefficients du modèle lasso, les valeurs absolues des coefficients du modèle cluster-lasso, et le nombre d'unitigs compris dans les 10 premiers clusters les plus prédictifs du modèle cluster-lasso ;
- la **figure 9** illustre, pour l'espèce *Salmonella* et pour l'antibiotique tétracycline, le ou les sous-graphes du graphe compacté, impliquant les motifs MAF étendus les plus prédictifs du modèle lasso (partie de gauche), et le ou les sous-graphes du graphe compacté impliquant les clusters les plus prédictifs du modèle cluster-lasso (partie de droite) ;
- la **figure 10** illustre, pour l'espèce *Salmonella* et pour l'antibiotique gentamicine, les valeurs absolues des coefficients du modèle lasso, les valeurs absolues des coefficients du modèle cluster-lasso, et le nombre d'unitigs compris dans les 10 premiers clusters les plus prédictifs du modèle cluster-lasso ;
- la **figure 11** illustre, pour l'espèce *Salmonella* et pour l'antibiotique gentamicine, le ou les sous-graphes du graphe compacté, impliquant les motifs MAF étendus les plus prédictifs du modèle lasso (partie de gauche), et le ou les sous-graphes du graphe compacté impliquant les clusters les plus prédictifs du modèle cluster-lasso (partie de droite) ;
- la **figure 12** illustre, pour l'espèce *Neisseria gonorrhoeae* et pour l'antibiotique céfixime, le ou les sous-graphes du graphe compacté, impliquant les motifs MAF étendus les plus prédictifs du modèle lasso (partie de gauche), et le ou les sous-graphes du graphe compacté impliquant les clusters les plus prédictifs du modèle cluster-lasso (partie de droite) ;
- la **figure 13** illustre, pour l'espèce *Neisseria gonorrhoeae* et pour l'antibiotique céfixime, les valeurs absolues des coefficients du modèle lasso, les valeurs absolues des coefficients du modèle cluster-lasso, et le nombre d'unitigs compris dans les 10 premiers clusters les plus prédictifs du modèle cluster-lasso ;

- la **figure 14** illustre pour l'espèce *Staphylococcus aureus* et pour l'antibiotique tétracycline, le ou les sous-graphes du graphe compacté, impliquant les motifs MAF étendus les plus prédictifs du modèle lasso (partie de gauche), et le ou les sous-graphes du graphe compacté impliquant les clusters les plus prédictifs du modèle cluster-lasso (partie de droite) ;
- la **figure 15** illustre, pour l'espèce *Staphylococcus aureus* et pour l'antibiotique tétracycline, les valeurs absolues des coefficients du modèle lasso, les valeurs absolues des coefficients du modèle cluster-lasso, et le nombre d'unitigs compris dans les 10 premiers clusters les plus prédictifs du modèle cluster-lasso ;
- la **figure 16** illustre un organigramme de détection d'une séquence génomique dans un génome ;
- la **figure 17** illustre la décomposition de la séquence génomique en k-mers ;
- la **figure 18** illustre des pourcentages de présence de k-mers dans le génome devant être atteints pour détecter la séquence génomique, pourcentages dépendant de longueur de ladite séquences ;
- les **figures 19A** et **19B** illustrent l'AUC d'une prédiction lasso et d'une prédiction cluster-lasso en fonction de la profondeur de séquençage d'isolats de souches de *Klebsiella pneumoniae;* et
- les **figures 20** et **21** illustre l'AUC d'une prédiction cluster-lasso en fonction de la profondeur de séquençage d'échantillon métagénomique comprenant des souches de *Klebsiella pneumoniae.*

## DESCRIPTION DETAILLEE DE L'INVENTION

### A. MODE DE REALISATION DE L'INVENTION

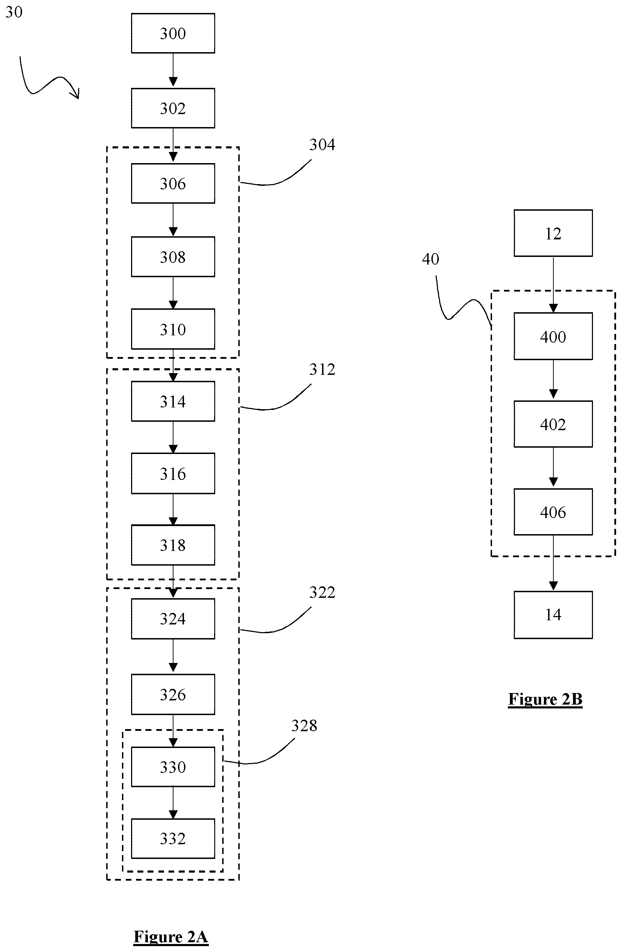

En se référant aux **figures 2A et 2B,** un procédé selon l'invention comporte une première partie **30** d'apprentissage d'un modèle de prédiction de la susceptibilité d'une souche bactérienne, appartenant à une espèce bactérienne donnée, à un antibiotique en fonction du génome bactérien de ladite souche, et une seconde partie **40** appliquant ledit modèle à une souche de ladite espèce bactérienne pour prédire sa susceptibilité inconnue.

La première partie **30** débute par l'établissement, en **300,** d'une base de données génomique et phénotypique pour ladite espèce. En particulier, un ensemble de souches est prélevé, par exemple sur des patients infectés par ladite espèce, et chaque souche prélevée est séquencée pour obtenir son génome complet, par exemple à l'aide d'une plateforme de séquençage de type MiSeq de la société Illumina, et un antibiogramme est établie pour connaître sa susceptibilité à l'antibiotique - résistant (« R »), intermédiaire (« I ») ou sensible (« S ») conformément aux concentrations critiques (ou « breakpoints » en anglais) de la norme CLSI ou de la norme EUCAST- par exemple à l'aide d'un Vitek 2 de la société bioMérieux. De préférence, les génomes prennent la forme de séquences assemblées (ou « contigs ») produites par l'assemblage numérique de séquences numériques produites par la plateforme de séquençage (ou « reads ») d'une manière connue en soi. Le génome numérique complet et la susceptibilité à l'antibiotique de chaque souche sont mémorisés dans une base de données informatique pour constituer un ensemble de données d'apprentissage et un ensemble de données de test.

De manière avantageuse, mais optionnelle, les états « résistant » et « intermédiaire » sont fusionnés de sorte à obtenir deux états de susceptibilité à l'antibiotique. De cette manière, il est défini un problème de classification binaire distinguant les souches bactériennes sensibles (« S ») des souches bactériennes non sensibles (« NS »). Par exemple, l'état S est codé par le chiffre 0 et l'état NS est codé par le chiffre 1.

Le reste de l'étape **30** est mis en œuvre par ordinateur et débute, en **302,** par le partitionnement en deux de la base de données, de manière à obtenir une base de données d'apprentissage et une base de données de test. De préférence, la partition est une partition dite « 10-fold », 9 dixièmes de la base constituant la base d'apprentissage et le dixième restant constituant la base de test. Le nombre total de souches bactériennes utilisées pour la base de données d'apprentissage est ci-après noté *N*.

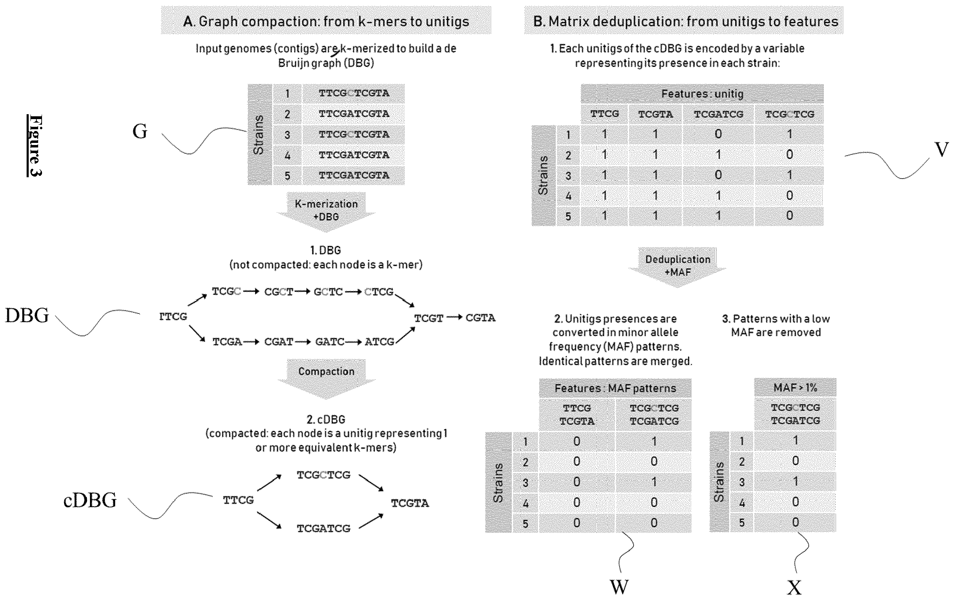

Dans l'étape **304** suivante, un ensemble de variables descriptives du génome bactérien est déterminé. En se référant à la **figure 3** en parallèle de la **figure 2,** les génomes **G** de la base de données d'apprentissage sont d'abord décrits en k-mers, en **306,** de taille *k* comprise entre 15 et 50, afin de capter de manière optimale la variabilité génétique de l'espèce bactérienne tout en limitant la redondance des k-mers, par exemple *k* = 31. Dans une étape **308** suivante, une transformation des k-mers en un ensemble de séquences génomiques distinctes, sans perte d'information, est mise en œuvre. A cet effet, les k-mers sont d'abord transformés en graphe de De Bruijn **DBG** d'ordre *k* et d'alphabet A, T, G, C. Le graphe **DBG** est ensuite transformé en graphe de De Bruijn compacté **cDBG** en regroupant automatiquement les chemins linéaires, et donc n'ayant pas de ramification. Les k-mers sont ainsi codés dans un graphe dont les nœuds, différents les uns des autres, correspondent à des séquences de longueur variable, ces nœuds (et de manière équivalente, ces séquences) étant appelés « unitigs ».

Dans une étape **310** suivante, les unitigs sont regroupés en motifs par déduplication, motifs dont, optionnellement, la fréquence allélique mineure (« minor allele frequency » en anglais, ou MAF) est supérieure à un seuil prédéterminé « S$_{MAF}$ » compris entre 98% et 99,5%, par exemple 99%. En particulier :

a. chaque unitig du graphe compacté **cDBG** est encodé par une variable binaire représentant la présence ou l'absence de l'unitig dans chaque génome de la base de données. Il est ainsi obtenu une matrice **V** telle que sa composante $V_{i,j}$ est égale à 1 si le j$^{ième}$ unitig est présent dans le i$^{ième}$ génome de la base de donnée et égal à 0 s'il est absent.

b. Chaque vecteur $V_{.,j}$ de la matrice **V**, i.e. chaque colonne associée à un unitig, est ensuite corrigée dans le but de calculer une MAF de l'unitig. Si la fréquence allélique du j$^{ième}$ unitig est supérieure à 0,5, signifiant qu'il est observé dans plus de 50% des génomes de la base de données (i.e. $\frac{1}{n}\sum_{i=1}^{n}V_{i,j} > 0,5$ ), alors la colonne $V_{.,j}$ est transformée de telle sorte que $\forall i$, $V_{i,j} = |1 - V_{i,j}|$. Cette transformation a pour avantage de rendre identique deux colonnes de la matrice **V** initialement complémentaires, de sorte que la co-occurrence de présence de unitigs est identique à leur co-occurrence d'absence.

c. la matrice **V** ainsi transformée subit ensuite un filtrage de ses colonnes identiques de sorte à regrouper en motifs les unitig de co-occurrence parfaite, c'est-à-dire présent des motifs d'absence/présence identiques dans les génomes de la base de données. Par exemple si la matrice **V** transformée a sa première colonne $V_{.,1}$ identique à sa seconde colonne $V_{.,2}$ l'une des colonnes est supprimée et la colonne restante code pour l'union des unitigs de la première et seconde colonne, formant ainsi un nouveau motif génomique ;

d. les motifs dont la fréquence est inférieure (100- S$_{MAF}$)%, i.e. pour S$_{MAF}$ = 99%, les motifs tels que $\frac{\sum_i V_{i,j}}{N} < 0,01$, sont ensuite éliminés, impliquant la suppression de la colonne correspondante de la matrice **V**. Il en résulte une matrice **X** telle que sa composante $X_{i,j}$ est égale à 1 si je j$^{ième}$ motif MAF est présent dans le i$^{ième}$ génome de la base de donnée et égal à 0 s'il est absent.

Les motifs restants, ci-après « motifs MAF », sont uniques, distincts les uns des autres, et sont ultérieurement utilisés comme variables d'outils d'apprentissage automatique décrits ci-après. Ce regroupement en motifs et le filtrage permettent de réduire de manière significative la redondance induite par la description des génomes en k-mers sans modifier la valeur informative intrinsèque des motifs MAF concernant leur implication éventuelle dans la susceptibilité à l'antibiotique. Les étapes **304-310** sont décrites plus en détails dans la thèse M. Jaillard Dancette, « Vers une cartographie des polymorphismes liés à la résistance aux antimicrobiens », 2018, et dans l'article de Jaillard M. et al. « A fast and agnostic method for genome-wide association studies : Bridging the gap between k-mers et genetic events », PLOS Genetics, 2018, et mises en œuvre par exemple à l'aide du logiciel DBGWAS développé par M. Jaillard (https://gitlab.com/leoisl/dbgwas).

La partie d'apprentissage **30** se poursuit par une étape **312** de regroupement des motifs MAF en variables de nombre limité, variables à la fois prédictive de la susceptibilité à l'antibiotique et ayant une signification biologique renforcée, ci-après « clusters ».

L'étape **312** débute, en **314,** par la sélection de motifs MAF ayant un pouvoir prédictif de la susceptibilité à l'antibiotique. De manière avantageuse, cette sélection est réalisée à l'aide d'un outil de régression logistique pénalisée de type LASSO. Plus particulièrement, pour chaque valeur $\lambda$ d'un ensemble de valeurs positives $\{\lambda_1,\lambda_2, ...,\lambda_m\}$, le problème d'optimisation suivant est résolu :

$$\widehat{\boldsymbol{\beta}}(\lambda) = arg \min_{\boldsymbol{\beta}\in\mathbb{R}^{p+1}} \sum_{i=1}^{N} \mathcal{L}\left(y_i, f\left(\boldsymbol{X}_{i,\cdot}\right)\right) + \lambda \sum_{j=1}^{p} |\beta_j| \qquad (1)$$

$$f\left(\boldsymbol{X}_{i,\cdot}\right) = \beta_0 + \sum_{j=1}^{p} \beta_j \boldsymbol{X}_{i,j} \qquad (2)$$

relations dans lesquelles :

- *p* est le nombre de motifs MAF, et donc le nombre de colonnes de la matrice *X*;
- *N* est le nombre de souches bactériennes utilisées pour constituer la base de données d'apprentissage, et donc le nombre de ligne de la matrice *X*;
- $y_i$ est la susceptibilité à l'antibiotique mesuré de la i$^{ème}$ souche bactérienne, associée à la ième ligne de la matrice *X*, à savoir $y_i$ = 0 si ladite souche est sensible et $y_i$ = 1 sinon ;
- $\mathcal{L}$ est une fonction de perte logistique, quantifiant la différence entre le phénotype mesuré $y_i$ et le phénotype prédit $f(X_{i,.})$, par exemple une différence au carré de ces deux termes ou une fonction de perte logistique, telle que

$$\mathcal{L}\left(y_i, f(X_{i,.})\right) = \log\left(1 - e^{y_i - f(X_{i,.})}\right).$$

Tout motif MAF activé pour l'une quelconque des valeurs de $\lambda$, et donc prédictif, est sélectionné, i.e. le j$^{ème}$ motif MAF est sélectionné si $\exists\, k \in \{1,2, ..., m\} \setminus \hat{\beta}_j(\lambda_k) \neq 0$. Comme cela est connu, les outils LASSO activent de manière incrémentale leurs variables le long du chemin de régularisation $\{\lambda_1, \lambda_2, ..., \lambda_m\}$, une ou plusieurs variables venant s'ajouter à celles déjà activées à mesure du parcours du chemin $\{\lambda_1, \lambda_2, ..., \lambda_m\}$, pour allumer un maximum de *N* variables. L'ensemble $\{\lambda_1, \lambda_2, ..., \lambda_m\}$ est avantageusement choisi de manière à obtenir une activation d'un maximum de motifs MAF. Par exemple, il est calculé selon la méthode décrite dans l'article de Friedman et al., « Regularization Paths for Generalized Linear Models via Coordinate Descent », Journal of Statistical Software, 2010, ou telle que mise en œuvre par exemple par le package *glmnet 3.0.2,* implémenté en R, et disponible par exemple à l'adresse https://cloud.r-project.org/web/packages/glmnet/index.html. Cette méthode permet notamment de choisir au préalable le nombre *m* de variables de régularisation $\lambda$. Ce nombre est par exemple choisi égal à 100.

Les motifs MAF sélectionnés sont ci-après nommés « motifs MAF actifs », le nombre de motifs MAF actifs étant noté $p_a$. Leurs rangs, notés $a_i$ (i.e. leurs indices de colonnes dans la matrice *X*), sont mémorisés dans un ensemble $a = \{a_1, a_2, ..., a_i, ..., a_{p_a}\}$.

L'étape **310** consiste ensuite, en **316**, à identifier les motifs MAF non sélectionnés à l'étape **314** mais qui présentent un taux de co-occurrence minimal dans les génomes avec les motifs MAF actifs, puis d'ajouter les motifs MAF ainsi identifiés à la liste des motifs MAF actifs. Le taux de co-occurrence de deux motifs est avantageusement mesuré par la corrélation entre leurs colonnes correspondantes de la matrice *X*. Pour ce faire, une matrice *G,* de dimensions $p_a \times p$, est calculée telle que :

$$\forall (i,j) \in [1, p_a] \times [1, p], G_{i,j} = cor\left(X_{.,a_i}, X_{.,j}\right) \qquad (3)$$

où *cor* est par exemple la corrélation linéaire de Bravais-Pearson, $X_{.,a_i}$ et $X_{.,j}$ désignant respectivement la $a_i$$^{ème}$ et la j$^{ème}$ colonnes de la matrice *X*.

Ensuite, tout motif MAF présentant une corrélation supérieure à un seuil $s_1$ prédéfini avec une motif MAF actif est sélectionné, et ajouté à la liste des motifs MAF actifs, l'ensemble des motifs MAF ainsi sélectionnés état nommé ci-après « motifs MAF actifs étendus ». En d'autres termes, le j$^{ème}$ motif MAF, $j \in [1,p]$, est sélectionné si $\exists\, i\, /\, G_{i,j} > s_1$. $s_1$ est un nombre qui permet de fixer le taux de co-occurence requis entre les motifs MAF actifs étendus. Il est compris entre 0,5 et 1, de préférence entre 0,8 et 1, et de manière avantageuse compris entre 0,9 et 0,95, par exemple 0,95. Le nombre total de motifs MAF actifs étendus, noté $p_e$, est alors très inférieur au nombre initial *p* de k-mers ($p_a < p_e \ll p$), de l'ordre de $10^3$ contre $10^5$ à $10^6$. Les rangs des motifs MAF actifs étendus, notés $e_i$ (i.e. leurs indices de colonnes dans la matrice *X*), sont mémorisés dans un ensemble $e = \{e_1, e_2, ..., e_i, ..., e_{p_e}\}$.

Dans une étape **318** suivante, des groupes, ou « clusters », de motifs MAF actifs étendus fortement co-occurents sont explicitement définis par la mise en œuvre d'un outil de partitionnement des données (ou « clustering analysis »). De préférence, un partitionnement hiérarchique (ou « hierarchical clustering ») est mis en œuvre à partir d'une matrice de distance calculée à partir des taux de co-occurrence entre les motifs MAF actifs étendus. Le partitionnement hiérarchique est par exemple celui décrit dans l'article de Bühlmann P. et al, « Correlated variables in regression : Clustering and sparse estimation », Journal of Statisical Planning and Inference, 2013, ou mis en œuvre parla fonction « *hclust* » du package *Stats* 3.6.2 implémenté en *R* et disponible par exemple à l'adresse https://www.rdocumentation.org/packages/stats/versions/3.6.2/source, en utilisant un critère d'agrégation basé sur la distance la plus proche (ou « single-linkage » en anglais).

Plus particulièrement, la matrice de distance utilisée par le partitionnement hiérarchique est une matrice *D,* de dimensions $p_e \times p_e$, calculée de la manière suivante, $X_{.,e_i}$ et $X_{.,e_j}$ désignant respectivement la $e_i$$^{ème}$ et la $e_j$$^{ème}$ colonnes de la matrice *X*:

$$\forall (i,j) \in [1, p_e] \times [1, p_e], \boldsymbol{D}_{i,j} = \left| 1 - cor\left( \boldsymbol{X}_{.,e_i}, \boldsymbol{X}_{.,e_j} \right) \right| \qquad (4)$$

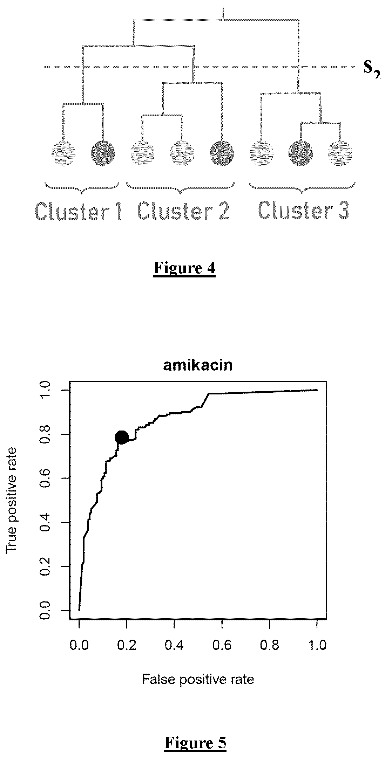

Il est ainsi obtenu un dendrogramme des motifs MAF actifs étendus. Ce dendrogramme est ensuite partitionné, à une hauteur 1 - $s_2$ comme cela est illustré à la **figure 4,** où $s_2$ est un nombre compris entre 0 et 1 fixant le taux de co-occurence dans les clusters, préférablement compris entre 0,5 et 1, de préférence entre 0,8 et 1, et de manière avantageuse compris entre 0,9 et 0,95, par exemple 0,95. La partie « inférieure » du dendrogramme définit ainsi des clusters dans lesquels les motifs MAF actifs étendus sont distribués en fonction de leur co-occurence. Chaque cluster est en outre unique et ne partage aucun motif MAF, et en particulier aucun unitig, avec un autre cluster. Les clusters, au nombre de $p_c$, sont notés $C_1$, $C_2$, ..., $C_j$ ... $C_{p_c}$ et chaque cluster $C_j$ est le regroupement de $p_j$ motifs MAF actifs étendus de rangs (i.e. leurs indices de colonnes dans la matrice $X$) compris dans l'ensemble $c_j$ = {$c_{j,1}$, $c_{j,2}$, ..., $c_{j,l}$, ..., $c_{j,p_j}$}.

Dans une étape **320** suivante, pour chaque cluster $C_j$, les unitigs le constituant sont décomposés en k-mers, avec $k$ compris entre 15 et 50, par exemple $k$ = 31, en faisant glisser, par pas de 1, une fenêtre de longueur $k$ sur les unitigs. Pour chaque cluster $C_j$, si on note $q_j$ le nombre d'unitigs le constituant, il est mémorisé $q_j$ ensembles de k-mers respectivement associés aux unitigs. Par soucis de clarté, les k-mers et les unitigs associés aux clusters sont nommés respectivement « k-mers de clusters » et « unitigs de clusters ».

Dans une étape suivante **322** suivante, il est mis en œuvre un apprentissage d'un modèle de prédiction de la susceptibilité à l'antibiotique dont les variables sont les clusters $C_1$, $C_2$, ..., $C_j$, ... $C_{p_c}$.

L'étape **322** débute, en **324,** par le calcul de la valeur de chaque cluster pour chaque génome de la base de données d'apprentissage. De manière avantageuse, cette valeur est égale à la moyenne des valeurs des motifs MAF le constituant. Il est ainsi obtenu une matrice $\boldsymbol{Y}$ de dimensions $N \times p_c$, telle que :

$$\forall j \in [1, p_c], \boldsymbol{Y}_{.,j} = \frac{1}{p_j} \times \sum_{l=1}^{p_j} \boldsymbol{X}_{.,c_{j,l}} \qquad (5)$$

En **326,** plusieurs modèles de prédiction de la susceptibilité à l'antibiotique sont appris à partir de la base de donnée d'apprentissage, l'étape **328** suivante consistant à sélectionner le plus performant selon des critères prédéterminés.

De manière avantageuse, les modèles de prédiction sont appris à l'aide de régression logistique pénalisée qui permettent de réduire le nombre de clusters prédictifs finalement retenus par le modèle tout en conservant un haut niveau de performance. En particulier, les modèles de prédiction sont des modèles selon les relations suivantes :

$$\begin{cases} \widehat{Su} = S \ si \ G(g) < S_p \\ \widehat{Su} = NS \ si \ G(g) \geq S_p \end{cases} \qquad (6)$$

$$G(g) = \hat{B}_0 + \sum_{j=1}^{p_c} \hat{B}_j \boldsymbol{Y}_{C_j} \qquad (7)$$

relations dans lesquelles :

- $\widehat{Su}$ est la susceptibilité prédite d'une souche bactérienne appartenant à l'espèce bactérienne ;
- $g$ est le génome de ladite souche ;
- $\hat{B}$ est un vecteur de paramètres de $\mathbb{R}^{p_c+1}$ ;
- $\boldsymbol{Y}_{C_j}$ est la valeur du $j^{ième}$ cluster $C_j$ pour le génome $g$ ;
- $S_p$ est un seuil prédéterminé positif.

L'étape **326** débute par l'apprentissage du modèle $G$ réalisé à l'aide d'un outil de régression logistique parcimonieuse sur la base de données d'apprentissage, e.g. une régression pénalisée de type LASSO. Un modèle G est notamment calculé pour chaque valeur $\lambda$ d'un ensemble de valeurs positives $\{\lambda_1^c, \lambda_2^c, \ldots, \lambda_i^c, \ldots, \lambda_M^c\}$ en résolvant un problème d'optimisation selon la relation :

$$\widehat{B}(\lambda) = arg \min_{B \in \mathbb{R}^{p_c+1}} \sum_{i=1}^{N} \mathcal{L}\left(y_i, G(Y_{i,.})\right) + \lambda \sum_{j=1}^{p_c} |B_j| \qquad (7)$$

relation dans laquelle :

- $y_i$ est la susceptibilité à l'antibiotique mesuré de la i$^{ème}$ souche bactérienne, associée à la ième ligne de la matrice $Y$, à savoir $y_i = 0$ si ladite souche est sensible et $y_i = 1$ sinon ;

- $\mathcal{L}$ est une fonction de perte logistique, quantifiant la différence entre le phénotype mesuré $y_i$ et le phénotype prédit $G(Y_{i,.})$, par exemple une différence au carré de ces deux termes ou une fonction de perte logistique, telle que

$$\mathcal{L}\left(y_i, f(X_{i,.})\right) = \log\left(1 - e^{y_i - f(X_{i,.})}\right).$$

Par exemple, il est calculé selon la méthode décrite précédemment en relation avec l'étape **314,** avec un nombre $M = 100$. Il est ainsi obtenu 100 modèles, noté $G_{\lambda_i^c}$ et donc 100 modèles de prédiction selon les relations (5)-(6), chacun dépendant de la valeur de seuil $S_p$, notée ci-après $G_{\lambda_i^c}(S_p)$.

Une estimation de la performance de chaque modèle de prédiction $G_{\lambda_i^c}(S_p)$, est ensuite calculée en **328** à partir de la base de données de test. L'évaluation de la performance permet en parallèle de calculer la valeur du seuil $S_p$.

En particulier, dans une étape **330,** pour chaque génome de la base de test, il est mise en œuvre :

a. une détection des k-mers de clusters présents dans le génome par homologie parfaite, c'est-à-dire qu'un k-mer est détecté s'il est présent à l'identique dans le génome ;

b. une détection qu'un unitig de cluster est présent dans le génome si le pourcentage de k-mers de cluster le constituant, déterminés comme présents dans le génome à l'étape précédente, est supérieur à un premier seuil de détection prédéterminé $s_{uni}$;

c. le calcul d'un indicateur de détection qu'un motif MAF étendu constitué de plusieurs unitigs de cluster est présent dans le génome, défini selon l'une ou l'autres des options suivantes :

i. l'indicateur est égal à 1 si le pourcentage d'unitigs de cluster le constituant, déterminés comme présents à l'étape précédente, est supérieur à un second seuil de détection prédéterminé $s_{clus}$, par exemple un seuil supérieur ou égal à 20%, par exemple 25%. Dans le cas contraire, l'indicateur est égal à 0. Cette option est celle appliquée pour les exemples décrits ci-après ; ou

ii. l'indicateur est égal à 1 si tous les unitigs de cluster le constituant sont déterminés comme présents à l'étape précédentes, et égal à 0 sinon ; ou

iii. l'indicateur est égal à 1 si au moins un unitig de cluster le constituant est déterminés comme présents à l'étapes précédentes, et égal à 0 sinon.

iv. l'indicateur est égal au pourcentage d'unitigs de clusters le constituants ayant été détectés comme présents à l'étape précédentes.

De manière avantageuse, le seuil de détection $s_{uni}$ dépend de la longueur de l'unitig de cluster. En particulier pour $k$ compris entre 15 et 50, par exemple $k = 31$, en notant $L$ la longueur d'un unitig de cluster pour lequel on cherche à connaitre la présence dans le génome, si $L \leq 61$ alors $s_{uni} = 90\%$, si $61 < L \leq 100$ alors $s_{uni} = 80\%$ et si $100 < L$ alors $s_{uni} = 70\%$.

L'étape **328,** en **330,** par le calcul de la valeur d'un cluster comme étant égale à la moyenne des indicateurs de détection des motifs MAF étendus le constituant, déterminés à l'étape précédente. On note que pour les options i, ii et iii, cette moyenne correspond au pourcentage de motifs MAF étendus détectés comme présent. Une fois les valeurs des clusters calculées pour tous les génomes de la base de test, l'étape **330,** se poursuit par une stratégie de sélection du modèle qui maximise la sensibilité, la spécificité et la parcimonie du modèle (i.e. minimise le nombre de clusters effectivement utilisés pour prédire la susceptibilité). Pour ce faire, pour chaque modèle $G_{\lambda_i^c}(S_p),$ il est fait varier le seuil $S_p$, et pour chaque valeur du seuil $S_p$ la sensibilité et la spécificité sont calculées selon les relation :

$$sensibilit\acute{e} = \frac{TP}{TP + FN} \qquad (7)$$

$$sp\acute{e}cificit\acute{e} = \frac{TN}{FP + TN} \qquad (8)$$

où TP, TN, FP, et FN sont respectivement les nombres de vrais positifs, vrais négatifs, faux positifs et faux négatifs décrits au tableau 1.

|  |  | Condition réelle $y_i$ | |
| --- | --- | --- | --- |
|  |  | NS | S |
| Prédiction $\widehat{Su}$ | NS | TP | FP |
|  | S | FN | TN |

Tableau 1

Les valeurs de la sensibilité et de (1 moins la spécificité) pour les valeurs du seuil $S_p$ sont ensuite reportés sur une courbe ROC, respectivement en ordonnée et abscisse, l'aire sous la courbe ROC, notée « AUC », est calculée et mémorisée.

Une valeur optimale $\widetilde{S_{p,i}}$ du seuil $S_p$ pour le modèle $G_{\lambda_i^c}(S_p)$ est alors calculée comme celle correspondant au point de la courbe ROC le plus proche du point d'abscisse 0 et d'ordonnée 1, comme illustré à la **figure 5.** La précision équilibrée (« balanced accuracy » en anglais, ou « bACC ») est ensuite calculée pour le modèle $G_{\lambda_i^c}(\widetilde{S_{p,i}})$ selon la relation suivante, la bACC, la sensibilité et la spécificité pour le modèle $G_{\lambda_i^c}(\widetilde{S_{p,i}})$ sont mémorisées :

$$bACC = \frac{sensibilit\acute{e} + sp\acute{e}cificit\acute{e}}{2} \qquad (9)$$

Le modèle finalement retenu parmi les modèle $G_{\lambda_i^c}(\widetilde{S_{p,i}})$ est le modèle le plus parcimonieux maximisant la bACC à une tolérance près, par exemple le modèle tel que :

a. $A$ est l'ensemble des modèles $G_{\lambda_i^c}(\widetilde{S_{p,\iota}})$ tels que leurs bACC est supérieur $max(bACC)$-0,01, ou $max(bACC)$ est le maximum des bACC calculées.

b. le modèle sélectionné est le plus parcimonieux des modèles de l'ensemble A.

Le modèle sélectionné est mémorisé dans une mémoire informatique pour une utilisation ultérieure **40** consistant à prédire la susceptibilité à l'antibiotique pour une souche bactérienne de l'espèce bactérienne. Les clusters retenu, et donc les unitigs les constituants, forment ainsi une signature génomique de la susceptibilité à l'antibiotique.
Notamment, cette prédiction consiste à :

a. séquencer, en **400,** le génome de la souche bactérienne en appliquant un séquence de génome complet, par exemple de la manière décrite en relation avec la figure 1 ;

b. calculer, en **402,** la valeur de chaque cluster du modèle de prédiction mémorisé de la manière décrite précédemment ;

c. calculer, en **404,** la susceptibilité $\widehat{Su}$ de la souche à l'aide des relations (5)-(6) pour le modèle mémorisé.

B. EXEMPLES

*B.1. Klebsiella pneumoniae*

Le procédé décrit aux **figures 2A** et **2B** a été mis en œuvre pour la prédiction de la susceptibilité à différents antibiotiques de l'espèce bactérienne *K. pneumoniae.* Le tableau 2 répertorie le nombre de souches utilisées pour l'apprentissage et la validation du modèle de prédiction, leurs phénotypes NS/S et les différents antibiotiques testés.
Le tableau 3 répertorie les performances du modèle appris conformément au procédé selon l'invention, nommé « modèle cluster-lasso » ou « cluster-lasso », et les performance d'un modèle de prédiction appris uniquement sur la base d'une régression logistique Lasso de l'état de la technique, nommé « modèle lasso » ou « lasso ». Les performances présentées dans ce tableau correspondent à une estimation par validation croisée, selon la procédure décrite précédemment. Pour ce dernier, des modèles selon les relations (1) et (2) sont calculés, des seuils $\widehat{Su}$ et un modèle final sélectionné de la manière décrite précédemment pour le procédé selon l'invention, et donc sur la base des mêmes critères de performance.
Les colonnes « support » indiquent le nombre de variables prédictives retenues pour le modèle de prédiction, i.e. le nombre de clusters pour lesquels $\hat{B}_j \neq 0$ pour le cluster-lasso et le nombre de « motifs MAF actifs » pour lesquels $\hat{\beta}_j$ pour le lasso. Les colonnes « unitigs » indiquent le nombre total d'unitigs retenus pour la signature génomique, avec entre parenthèses le nombre d'unitigs dans la plus large des variables prédictives.

Tableau 2

| | Base de données d'apprentissage | | Base de données de test | |
|---|---|---|---|---|
| | NS | S | NS | S |
| amikacine | 346 | 1319 | 191 | 160 |
| aztréoname | 1426 | 216 | 250 | 10 |
| céfépime | 961 | 608 | 235 | 53 |
| céfoxitine | 976 | 667 | 319 | 138 |
| céftazidime | 1259 | 136 | 457 | 125 |
| ciprofloxacine | 1461 | 201 | 471 | 137 |
| imipenème | 504 | 1160 | 259 | 301 |
| méropénème | 524 | 1134 | 297 | 86 |

(suite)

|  | Base de données d'apprentissage | | Base de données de test | |
|---|---|---|---|---|
|  | NS | S | NS | S |
| piper.tazo | 1228 | 432 | 382 | 146 |
| tétracyline | 928 | 737 | 273 | 155 |

Tableau 3

|  | lasso | | | | Cluster-lasso | | | |
|---|---|---|---|---|---|---|---|---|
|  | bACC | AUC | support | unitigs | bACC | AUC | support | unitigs |
| amikacine | 92,7 | 95,4 | 16 | 22(4) | 92,3 | 95,7 | 11 | 93(36) |
| aztréoname | 76,7 | 81,9 | 31 | 45(3) | 76,9 | 82,3 | 28 | 425(125) |
| céfépime | 74,0 | 80,4 | 53 | 65(3) | 73,6 | 79,8 | 34 | 385(111) |
| céfoxitine | 82,4 | 88,7 | 134 | 155(5) | 82,2 | 88,8 | 171 | 1052(221) |
| céftazidime | 91,6 | 95,8 | 51 | 69(5) | 90,7 | 95,3 | 43 | 863(185) |
| ciprofloxacine | 95,6 | 98,6 | 25 | 27(2) | 95,5 | 98,6 | 35 | 422(139) |
| imipenème | 93,1 | 93,6 | 10 | 10(1) | 92,7 | 93,4 | 7 | 241(194) |
| méropénème | 91,7 | 94,0 | 8 | 8(1) | 91,4 | 93,5 | 3 | 164(159) |
| piper.tazo | 81,6 | 89,6 | 127 | 144(4) | 81,5 | 89,0 | 120 | 1220(226) |
| tétracyline | 83,0 | 88,5 | 181 | 198(3) | 82,9 | 87,7 | 109 | 640(104) |

Comme on peut le noter, les performances du modèle cluster-lasso sont analogues à celle d'un modèle de prédiction dont les variables d'apprentissage ne sont pas contraintes par regroupement. On note ainsi que les deux modèles présentent des performances similaires en termes de précision équilibrée bACC et d'AUC, confirmant que la prise en compte ou non de la corrélation entre les caractéristiques a un impact limité en termes de performances prédictives. On note également que le support du modèle est souvent légèrement plus petit avec le cluster-lasso (pour 8 antibiotiques sur 10), ce qui suggère que plusieurs caractéristiques sélectionnées séparément avec le lasso ont fini par fusionner en un seul cluster par le cluster-lasso. Comme prévu, le nombre total d'unitigs impliqués dans un modèle cluster-lasso est significativement plus important. On note que ce nombre n'est pas réparti également entre les clusters prédictifs. Par exemple, dans le modèle de prédiction de la susceptibilité au méropénème, 159 sur les 164 unitigs sont présents dans un seul cluster, ce qui suggère la présence d'un gène comme caractéristique génomique prédictive.

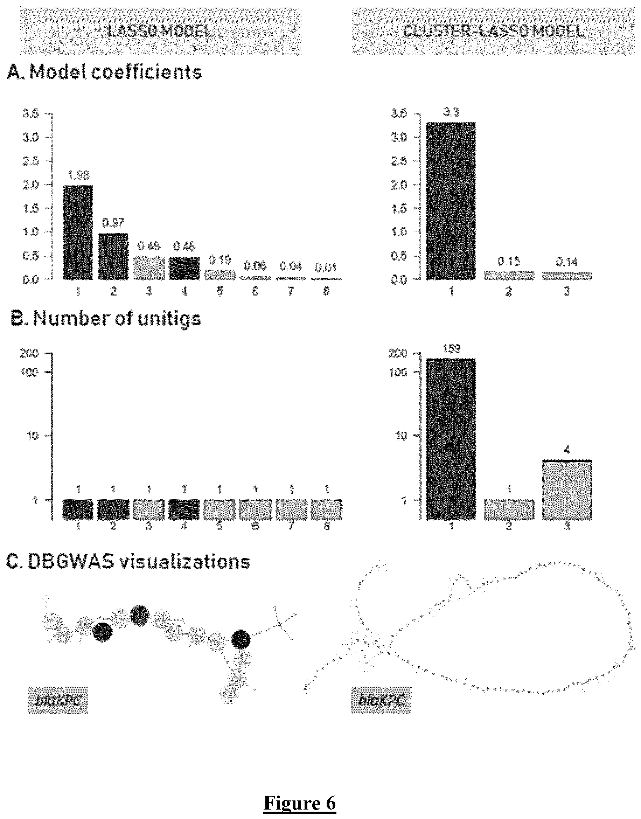

La **figure 6(A)** montre l'ampleur des coefficients des modèles pour le méropéneme. Comme cela est apparent, la signature du modèle cluster-lasso est essentiellement résumée par une seule caractéristique importante, tandis que 4 à 5 caractéristiques de la signature lasso ont un poids non négligeable. Il s'avère que le cluster avec le plus de poids prédictif est également le plus grand cluster en nombre d'unitigs. La visualisation de ce cluster dans le graphe de De Bruijn compacté cDBG (e.g. au moyen du logiciel DBGWAS, décrit précédemment), telle qu'illustrée à la **figure 6(C),** montre que les unitigs de ce cluster forment un long chemin linéaire dans le graphe. Cela suggère donc que ce cluster correspond à un gène entier. L'annotation de ce chemin linéaire, fournie par le logiciel DBGWAS, indique qu'il correspond au gène *blaKPC,* par ailleurs bien documenté dans la littérature pour son rôle dans la résistance au méropénème. La visualisation obtenue pour la signature lasso indique à contrario que 3 des 8 variables prédictives - les variables 1, 2 et 4 - sont également colocalisées dans une région annotée comme étant le gène *blaKPC.* Toutefois, le fait que le lasso ait sélectionné ces unitigs spécifiques au sein du gène *blaKPC* suggère que les déterminants de la résistance impliqués sont des mutations ponctuelles de ce gène, à savoir des SNP ou les indels. Si l'annotation au niveau du gène est la même que celle obtenue avec le cluster-lasso, l'interprétation de la signature en termes de variantes génétiques est donc radicalement différente. Un examen plus approfondi de la signature du lasso révèle que les 3 variables localisées dans le gène *blaKPC* sont en fait fortement corrélées. En détectant, selon l'invention, explicitement que ces entités sont corrélées et en les fusionnant dans un cluster, ainsi que d'autres unités génomiques corrélées qui ne sont même pas impliquées dans la signature du lasso, le cluster-lasso conduit donc à une interprétation plus significative biologiquement du modèle de prédiction sous-jacent, et ce à deux égards. Premièrement, en ce qui concerne la nature du déterminant génomique impliqué: acquisition ou mutation au sein d'un gène. Deuxièmement, en ce qui concerne sa contribution globale à prédiction de la susceptibilité, en sommant les contributions de plusieurs caractéristiques distinctes mais corrélées impliquées dans la signature du lasso.

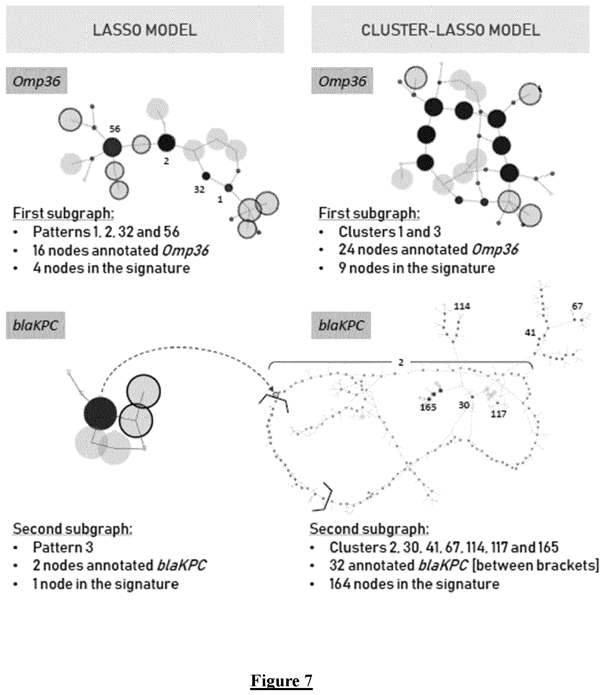

De manière similaire, la **figure 7** illustre l'interprétabilité des deux modèles de prédiction pour la céfoxitine. En se focalisant sur les sous-graphes du graphe cDGB dans lesquels sont localisés les deux clusters les plus prédictifs, l'annotation de ces deux régions identifie les mêmes gènes de résistance pour les deux méthodes (premièrement, le gène *OmpK36,* connu pour être impliqué dans les pompes à efflux, et deuxièmement le gène *blaKPC*). A contrario la nature du déterminant génomique (présence de gène, SNP, indel,...) ne peut être déduite de la signature du lasso.

L'interprétabilité peut être même très détaillée. Par exemple, en ce qui concerne le sous-graphe annoté *OmpK36* obtenu pour la signature cluster-lasso (panneau supérieur droit de la **figure 7**), il comprend 2 clusters (clusters 1 et 3), regroupant 9 unitigs. Ces unitigs présentent une topologie attribuable à un polymorphisme local, à savoir une bulle complexe, avec une fourche séparant les souches sensibles et résistantes comme décrit dans l'article de Jaillard M. et al. « A fast and agnostic method for genome-wide association studies : Bridging the gap between k-mers et genetic events », PLOS Genetics, 2018. En revanche, le sous-graphe correspondant obtenu pour le lasso (panneau en haut à gauche de la **figure 7**) comprend 4 motifs (motifs 1, 2, 32 et 56) avec 4 valeurs distinctes de $\hat{\beta_j}$. Les valeurs distinctes $\hat{\beta_j}$ peuvent conduire à des conclusions erronées concernant l'importance individuelle des séquences unitig correspondant. En effet, lorsque l'on regarde un alignement multiple intégrant des séquences annotées supplémentaires de *OmpK36,* il apparaît que les motifs MAF actifs 2 et 56 représentent le type sauvage et que les motifs 1 et 32 s'alignent sur la même insertion de deux acides aminés dans la boucle L3, comme décrit dans l'article Novais A. et al, « Spread of an OmpK36-modified ST15 Klebsiella pneumoniae variant during an outbreak involving multiple carbapenem-resistant Enterobacteriaceae species and clones », European Journal of clinical microbiology and infectious deseases, 2012. L'invention offre au contraire des valeurs B$_j$ moyennes pour chaque haplotype

Le deuxième sous-graphe obtenu pour la signature lasso (panneau en bas à gauche de la **figure** 7) ne comprend qu'une seule caractéristique de la signature (représentée en noir) et sept nœuds environnants (représentés en gris), dont deux sont annotés *blaKPC.* Comme le nœud unique de la signature n'est pas lui-même annoté, le sous-graphe pourrait être interprété comme un polymorphisme local dans la région promotrice du gène *blaKPC.* Cependant, le sous-graphe cluster-lasso (panneau en bas à droite de la **figure 7**) indique que ce seul unitig a été sélectionné par le lasso parmi des centaines d'unitigs hautement corrélés: ils appartiennent tous au cluster 2, qui comprend le gène complet *blaKPC* (illustré entre les parenthèses) et les séquences du plasmide dans lequel il est inséré et qui sont fortement co-occurentes aux séquences du gène.

Les informations complémentaires fournies par le cluster-lasso permettent ainsi de conclure que la première variable causale de la résistance à la céfoxitine est une mutation locale sur le gène *OmpK36.* Avantageusement, les technologies moléculaires (PCR, NGS,...) de prédiction de résistance à la céfoxitine cibleront spécifiquement cette mutation. De plus, la deuxième variable causale est l'acquisition d'un gène complet *blaKPC,* et toute séquence d'ADN spécifique à *blaKPC* pourra être avantageusement utilisée par de telles technologies pour prédire la résistance à la céfoxitine.

D'autres couples espèce bactérienne/antibiotique ont été testés. Sans entrer dans un niveau de détails en relation avec *Klebsiella pneumoniae,* ce qui suit décrit pour les espèces *Salmonella, Staphylococcus aureus,* et *Neisseria gonorrhoeae*:

- un premier et second tableaux similaires aux tableaux 2 et 3 précédents respectivement ;
- un première, deuxième et troisièmes figures illustrant respectivement, pour l'antibiotique considéré, les valeurs absolues des coefficients du modèle lasso, les valeurs absolues des coefficients du modèle cluster-lasso, et le nombre d'unitigs compris dans les 10 premiers clusters les plus prédictifs du modèle cluster-lasso ;
- une figure illustrant :

  ○ dans sa partie gauche, le ou les sous-graphes du graphe compacté **cDBG,** impliquant les motifs MAF étendus les plus prédictifs du modèle lasso. Les sous-graphes sont d'abord identifié par les motifs les plus prédictifs et lorsque d'autres motifs sont présents dans le sous-graphes, ils sont également représentés;
  ○ dans sa partie droit, le ou les sous-graphes du graphe compacté **cDBG** impliquant les clusters les plus prédictifs du modèle cluster-lasso. Les sous-graphes sont d'abord identifié par les clusters les plus prédictifs et lorsque d'autres clusters sont présents dans le sous-graphes, ils sont également représentés.

*B.2. Salmonella*

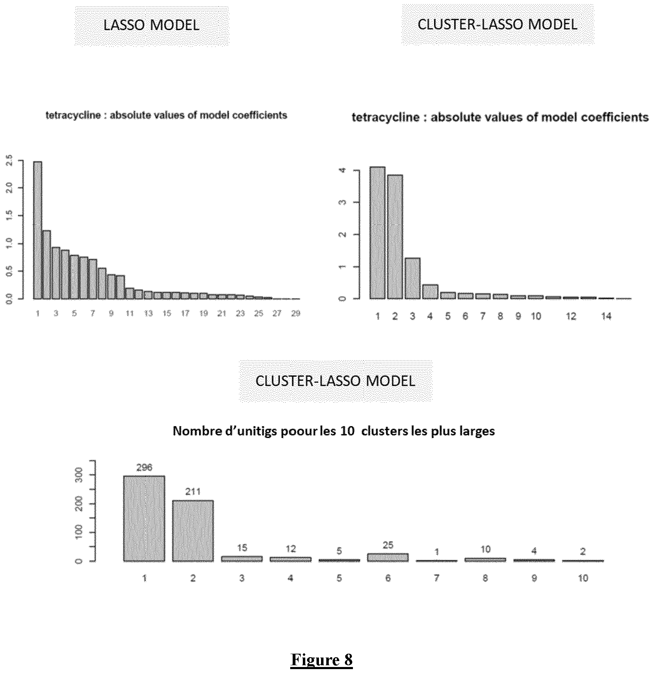

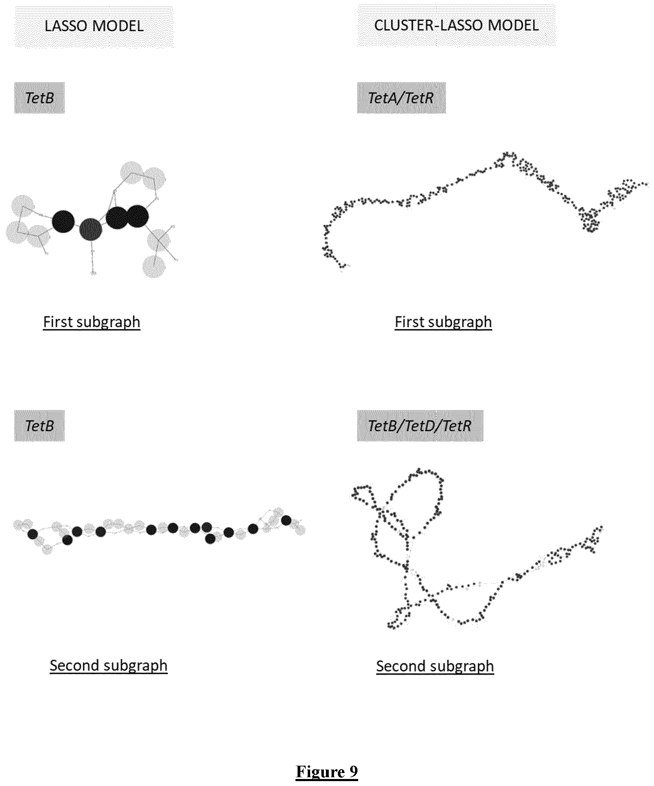

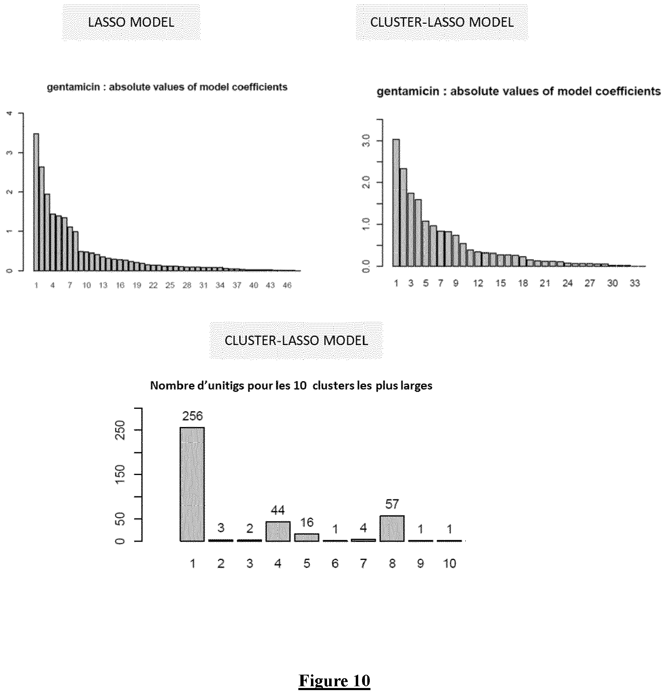

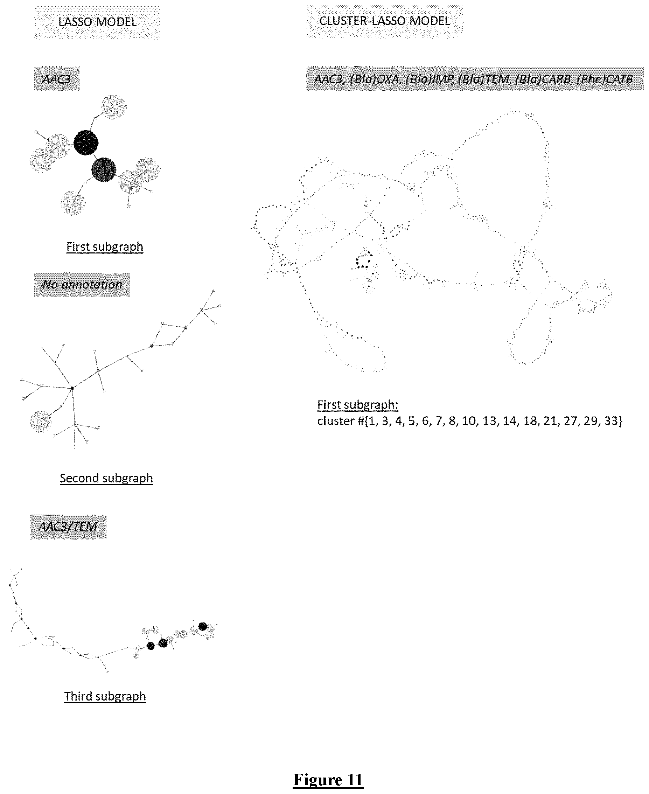

Tableaux 4 et 5, **figures 8** et **9** pour la tétracycline, **figures 10** et **11** pour la gentamicine.

A la différence du modèle lasso, qui identifie un possible ensemble de mutations ponctuelles dans les gènes *TetB* pour l'acquisition d'un résistance à la tétracycline, sans que cela soit par ailleurs conclusif au vu du nombre important de mutations que cela impliquerait, l'invention à base de cluster-lasso identifie l'acquisition de la résistance pour la présence du gène *TetA* (cluster 1), des gènes *TetB*/*TetD* (cluster 2), ainsi que l'acquisition du gène *TetR*.

Concernant la résistance à la gentamicine, l'invention conclut à l'acquisition du gène *AAC3* (cluster 1) et l'implication des gènes *OXA, IMP, TEM* dans les mécanismes de résistance, là où le modèle lasso échoue à identifier les gènes *OXA, IMP*.

Tableau 4

|  | Base de données d'apprentissage | |
| --- | --- | --- |
|  | NS | S |
| tétracycline | 2597 | 1901 |
| gentamicine | 637 | 3862 |

Tableau 5

|  | lasso | | | Cluster-lasso | | |
| --- | --- | --- | --- | --- | --- | --- |
|  | bACC | AUC | support | bACC | AUC | support |
| tétracycline | 97,4 | 98,2 | 29 | 97,1 | 98,1 | 15 |
| gentamicine | 96,8 | 98,2 | 48 | 96,5 | 98,2 | 34 |

*B.3. Neisseria gonorrhoeae*

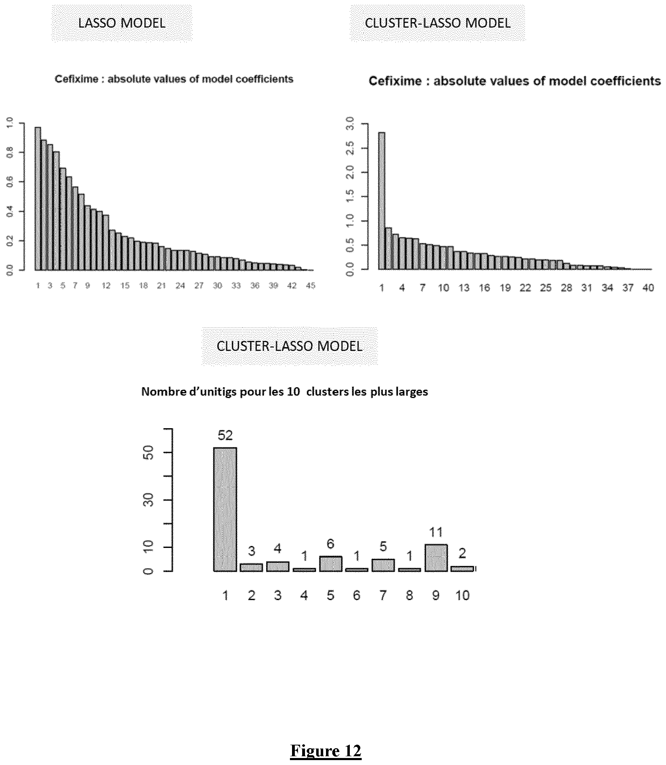

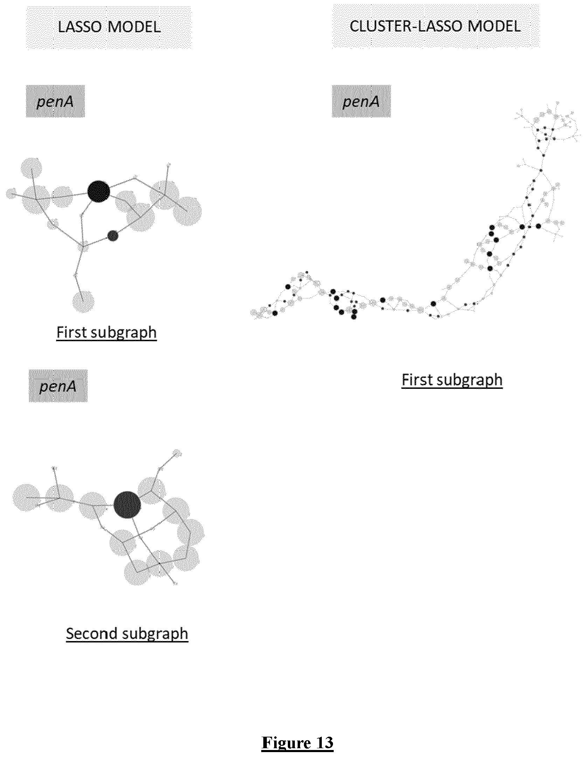

Tableaux 6 et 7, **figures 12** et **13**.

Concernant la résistance à la céfixime de *N. gonorrhoeae,* l'invention permet d'identifier l'acquisition de plusieurs recombinaison dans le gène *penM.*

Tableau 6

|  | Base de données d'apprentissage | |
| --- | --- | --- |
|  | NS | S |
| céfixime | 110 | 554 |

Tableau 7

|  | lasso | | | Cluster-lasso | | |
| --- | --- | --- | --- | --- | --- | --- |
|  | bACC | AUC | support | bACC | AUC | support |
| céfixime | 91,7 | 97,2 | 45 | 92,1 | 97,5 | 40 |

*Staphylococcus aureus*

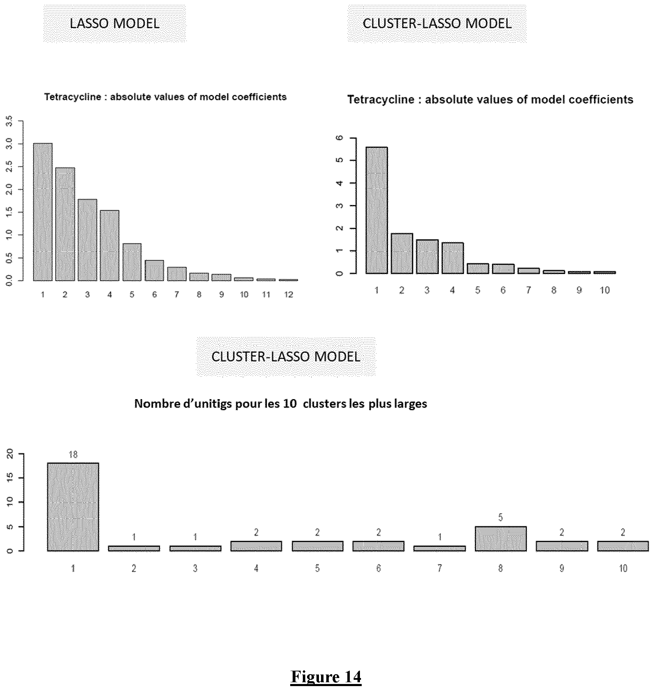

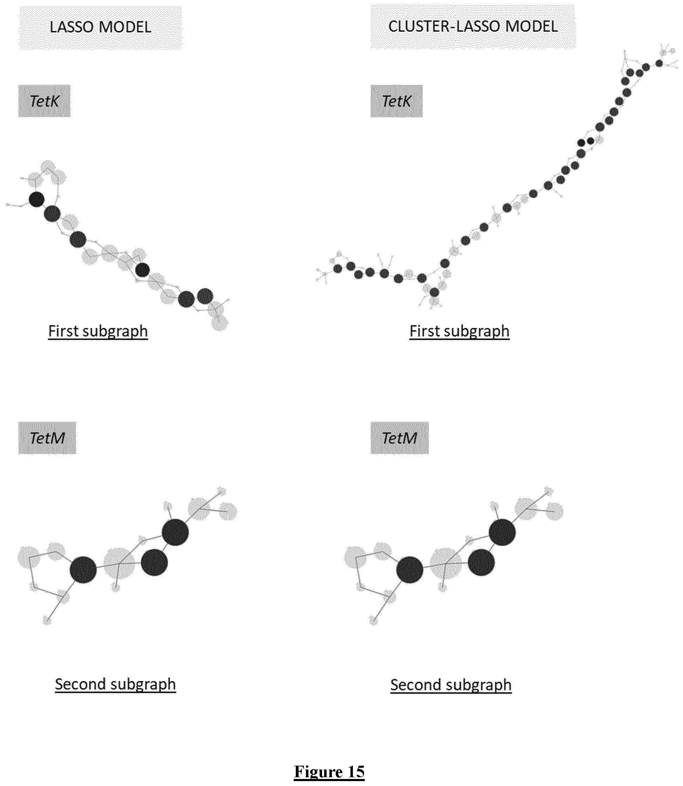

Tableaux 8 et 9, **figures 14** et **15**.

Concernant la résistance à la tétracycline de *S. aureus,* l'invention permet d'identifier l'acquisition du gène *TetK* (cluster 1), mais écarte l'acquisition du gène *TetM* (cluster 2 et 3) comme étant un déterminant génomique hautement prédictif de la résistance à la gentamicine au regard de la faiblesse des coefficients des clusters impliqués, à la différence du modèle lasso qui interprète le gène *TetM* comme hautement prédictif.

Tableau 8

| | Base de données d'apprentissage | |
|---|---|---|
| | NS | S |
| tétracycline | 27 | 468 |

Tableau 9

| | lasso | | | Cluster-lasso | | |
|---|---|---|---|---|---|---|
| | bACC | AUC | support | bACC | AUC | support |
| tétracycline | 96,9 | 96,7 | 12 | 98,9 | 99,6 | 10 |

C. MOYENS INFORMATIQUES DE MISE EN ŒUVRE DE L'INVENTION

Les étapes **302, 304, 312, 320,** tout comme les étapes **60** et **80** décrites ci-après, sont mises en œuvre par ordinateur, par exemple une unité informatique comprenant un ou plusieurs processeurs, d'espace de stockage et de mémoires vives, aptes à mémoriser des instructions informatiques qui, lorsqu'elles sont exécutées, réalisent les calculs décrits précédemment. L'unité informatique est par exemple un ordinateur personnel, un serveur, ou encore un cluster de calcul. De même, les étapes **402, 404** sont mises en œuvre par ordinateur, par exemple une unité informatique telles que précédemment décrite. L'unité des étapes **302, 304, 312, 320** et celle des étapes **402, 404** sont différentes ou sont une seule et même unité. De manière avantageuse, la susceptibilité prédite est affiché sur un écran d'ordinateur, mémorisée dans le système informatique d'un laboratoire ou d'un hôpital pour compléter le dossier d'un patient lorsque la souche bactérienne infecte un patient, ou sont transmis à un dispositif mobile d'un clinicien, par exemple un smartphone.

D. EXTENSION DE L'ENSEIGNEMENT DU MODE DE REALISATION DE L'INVENTION

D.1. Concernant la détection de la présence de k-mers, d'unitigs et de motifs MAF dans le génome bactérien - étape 330

Il a été décrit à l'étape **330** une manière de détecter la présence ou l'absence d'une séquence génomique, en particulier un unitig, ou d'un ensemble de séquences génomiques dans un génome bactérien, en particulier un ensemble de motifs regroupant des unitigs. D'une manière générale, le mode de réalisation répond à la problématique de savoir si une séquence ou un ensemble de séquences doit être détecté à l'identique dans le génome ou bien s'il est possible d'accepter un certain niveau de différence entre la séquence ou le groupe de séquences et les séquences ou groupes de séquences du génome pour conclure à leur présence ou absence. Notamment, comme expliqué en préambule, une homologie parfaite suppose que les données d'apprentissage soient complètes pour englober toute la variabilité de l'espèce biologique, ce qui est *de facto* difficile, notamment au regard de la plasticité de leur génome.

De plus, le génome séquencé peut être entaché d'erreur, notamment lorsqu'il est sous forme de « reads », c'est-à-dire de séquences produites en sortie des plateformes de séquençage avant tout traitement bioinformatique tel que l'assemblage par consensus ou le filtrage des reads de mauvaise qualité. Dans ce cas, une séquence, bien que présente dans le génome, peut être détectée comme absente en raison des erreurs de séquençage et *vice versa.* En particulier, le traitement bioinformatique comprend généralement le filtrage des reads de faible qualité, et optionnellement l'assemblable par consensus des reads filtrés de manière à obtenir des séquences assemblées, ou « contigs ». Le caractère optionnel de l'assemblage dépend généralement du contexte dans lequel est réalisée l'analyse de l'échantillon. L'assemblage a pour effet de réduire de manière très importante les erreurs de séquençage dans les contigs, à ce jour à un niveau de $10^{-5}$ pour les technologies SBS mises en œuvre dans les plateformes de la société Illumina Inc., et à niveau de $10^{-2}$ pour les technologies nanopores mises en œuvre dans les plateformes de la société Oxford Nanopore Technologies Ltd. Par contre l'assemblage nécessitant de grandes puissances de calcul et du temps, il est peu adapté à des applications génomiques de type « POC » (« point of care ») dont l'environnement informatique est généralement peu puissant et/ou à des applications rapides, voire temps réel. Dans ce contexte, l'analyse génomique, par exemple pour déterminer l'identité de la ou des espèces présentes dans l'échantillon et/ou leur(s) susceptibilité à un ou plusieurs antibiotiques(s) tel que décrit précédemment, est mis en œuvre directement sur les reads, filtrés ou non. Or les erreurs de séquençage sont de l'ordre de 2 à 3% pour les technologies SBS et jusqu'à 12% pour les technologies nanopores. Sans précaution particulière, l'analyse génomique peut mener à un taux d'erreur également important.

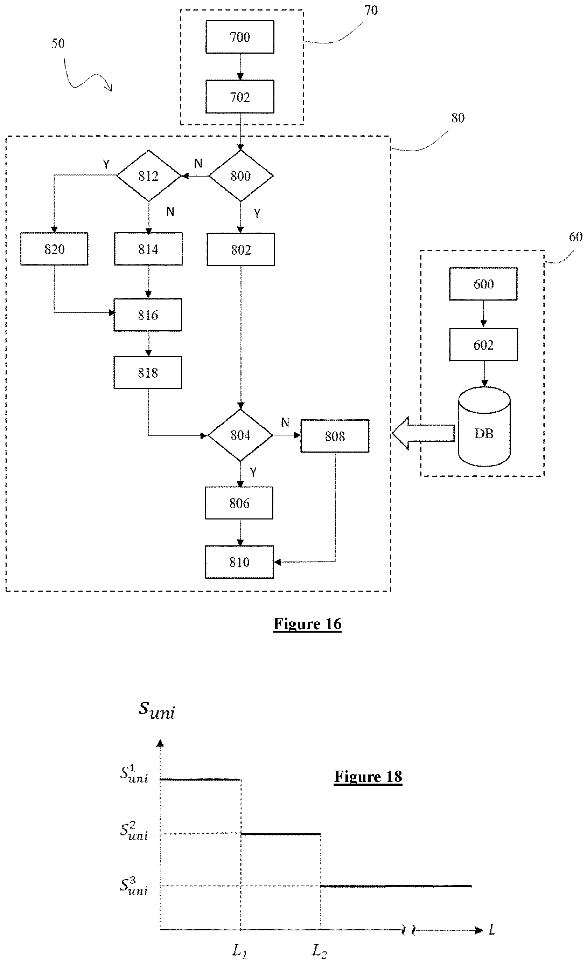

La figure 16 illustre un procédé **50** visant à détecter de manière plus robuste la présence ou l'absence d'une séquence génomique dans un génome de microorganisme, notamment d'une souche bactérienne, d'une souche de levure ou d'une souche de moisissure, afin de tenir compte de la variabilité génomique et des erreurs de séquençage. Ce procédé, bien qu'indépendant en soi des procédés des figures 2A et 2B, est avantageusement mis en œuvre à l'étape **330** et/ou à l'étape **402** de ceux-ci.

Le procédé **50** comporte une étape **70** de séquençage d'un échantillon comprenant une ou plusieurs souches de microorganisme, dans ce qui suit, et à titre d'exemple uniquement, une ou plusieurs souches bactériennes, et de prétraitement des reads produits par la plateforme de séquençage, et une étape **80** de détection d'une séquence génomique prédéterminée dans le génome d'une des souches bactériennes. Cette étape **80** comporte, optionnellement, la détection d'au moins un ensemble prédéterminé de séquences génomiques dans ledit génome.

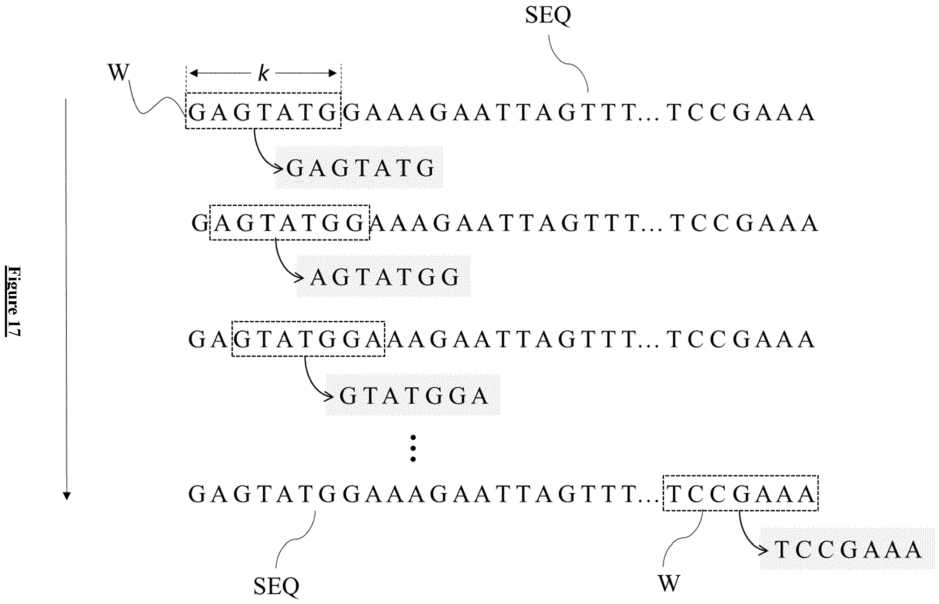

L'étape **80** utilise une décomposition de la séquence génomique, ainsi qu'un certain nombre de paramètres, calculés lors d'une étape **60,** par exemple mise en œuvre préalablement au procédé **50,** et mémorisés dans une base de données DB. Plus particulièrement, en se référant à la figure 17, l'étape **60** débute par une étape **600** dans laquelle la séquence génomique, notée « SEQ », est décomposée en k-mers de longueur constante $k,$ avec $k$ compris entre 15 et 50, par exemple $k = 31$, en faisant glisser, par pas constant, préférentiellement avec un pas de 1, une fenêtre W de longueur $k$ sur la séquence SEQ. A chaque position de la fenêtre W, un k-mer est ainsi mémorisé. Pour une séquence SEQ de longueur $L$, il est ainsi produit $(L - k + 1)$ k-mers. Dans une étape **602** optionnelle suivante, l'ensemble des k-mers produits est filtré de ses éventuels doublons pour ne conserver qu'un ensemble constitué de k-mers uniques, noté $KM = \{km_1, ....km_i, ..., km_S\}$, cet ensemble formant la décomposition de la séquence SEQ mémorisée dans la base DB.

L'étape **70** consiste, comme cela est connu en soit, et par exemple décrit en relation avec la figure 1 :

- dans une étape **700** à préparer l'échantillon pour le séquençage de l'ADN contenu dans l'échantillon et à séquencer l'ADN préparer, de sorte à produire et mémoriser des reads ;
- dans une étape **702** à réaliser un traitement bioinformatique comprenant généralement le filtrage des reads de faible qualité, et optionnellement l'assemblable par consensus des reads filtrés de manière à obtenir et mémoriser des séquences assemblées, ou « contigs ».

L'étape **80** de détection de la séquence génomique SEQ débute par un premier test, en **800,** consistant à savoir si cette détection est réalisée sur des contigs ou des reads. Si la détection est réalisée sur des contigs, à savoir des séquences génomiques dont le taux d'erreur est suffisamment faible pour permettre l'utilisation d'une homologie parfaite par la détection des k-mers, le procédé se poursuit, en **802,** par la détection de la présence ou de l'absence de chaque k-mer $km_i$ de l'ensemble $KM$ dans les contigs. En particulier, le k-mer $km_i$ est détecté s'il est présent à l'identique dans au moins un des contigs.

Dans une étape suivante **804,** un test est réalisé pour savoir si la séquence SEQ doit être détectée à l'identique dans le génome bactérien. Si tel est le cas, la séquence SEQ est détectée, en **806,** si la totalité des k-mer $km_i$ de l'ensemble KM sont détectés comme présents dans le contigs. On note à cet égard qu'en raison de la technologie de séquençage et/ou de la technologie d'assemblage, la séquence SEQ ne se retrouve pas nécessairement entière dans un contig, mais peut au contraire être fractionnée entre plusieurs contigs, la probabilité d'un tel fractionnement augmentant avec la longueur $L$ de la séquence SEQ. La décomposition en k-mers permet donc d'identifier cette dernière dans le génome même s'elle n'est pas présente en tant que telle dans les contigs.

Si, en **804,** il n'est pas recherché la séquence SEQ à l'identique, il est recherché dans le génome, en **808,** s'il existe au moins la séquence SEQ ou l'un de ses variants. Ces variants correspondent par exemple à des mutations de la séquence SEQ d'origine ou encore à une identification imparfaite de cette dernière. Comme décrit ci-dessus, la séquence SEQ peut être le produit d'une identification d'un déterminant génétique (e.g. de résistance, de virulence, d'identité...) se fondant sur des données ou des connaissances a priori. Si ces dernières sont imparfaites, la séquence SEQ peut ne pas tenir compte de toute la diversité réelle dudit déterminant. En autorisant l'identification de la séquence SEQ ou de l'un de ses variants, le procédé permet de corriger l'imperfection initiale des données et des connaissances et ainsi de détecté le déterminant génétique. Dans une moindre mesure, cela permet également de tenir compte d'éventuelles erreurs résiduelles de séquençage dans les contigs. Par ailleurs, indépendamment de la correction d'erreur, cela permet également, à partir d'une seule séquence SEQ, de détecter si au moins un membre d'un ensemble de variants est présent dans le génome, sans avoir à détecter de manière parfaite chacun desdits variants.

Dans une première variante, en **808,** la séquence SEQ ou l'un de ses variants est détecté si le pourcentage de ses k-mers constitutifs est supérieur à un seuil prédéterminé $s_{uni}$, par exemple supérieur à 70%. Dans une variante préférée, ce pourcentage est dépendant de la longueur $L$ de la séquence SEQ et plus particulièrement est décroissant en fonction de $L$. En effet, il est préférable de garder une longueur de k-mers suffisamment importante, en particulier supérieure à 15, et de préférence supérieure à 30, pour que ces derniers restent spécifiques de la séquence SEQ. Ainsi, à mesure que la longueur $L$ diminue, une différence avec la séquence SEQ se retrouve dans un pourcentage de plus en plus important de k-mers, ce que permet de corriger pourcentage $s_{uni}$ décroissant en fonction de la longueur $L$. Comme illustré à la figure 18, le pourcentage $s_{uni}$ est par exemple décroissant par morceau, et comprend trois valeurs.

Avantageusement, pour *k* compris entre 15 et 50, par exemple *k* = 31, si $L \le 61$ alors $s_{uni}$ = 90%, si $61 < L \le 100$ alors $s_{uni}$ = 80% et si $100 < L$ alors $s_{uni}$ = 70%.

Une fois la séquence SEQ détectée, de façon optionnelle, le procédé se poursuit, en 810, par la détection d'un ensemble de séquences génomiques, noté {$SEQ_1$, ..., $SEQ_i$, ... $SEQ_E$}, d'une manière décrite ci-après.

Si la détection de la séquence SEQ est réalisée sur des reads (test **800**), et donc avant tout traitement bioinformatique corrigeant les erreurs de séquençage, le procédé tient compte de ces dernières pour détecter avec précision la présence/absence d'un k-mer dans les reads, et donc dans le génome. L'avantage de détecter directement à partir des reads réside dans la rapidité du traitement de données, inférieur à 2-3 minutes pour un environnement de calcul donnée, alors que l'assemblage peut nécessiter à lui seul plus d'une heure pour le même environnement.

Dans une première variante, un k-mer est détecté si les reads contiennent un nombre minimal de copie dudit k-mer, par exemple un nombre supérieur ou égal à 3. Cette variante présente cependant le désavantage de ne pas tenir compte de la profondeur de séquençage (« sequencing depth of coverage » en anglais). En première approximation, les erreurs de séquençage sont réparties sur tout le génome de sorte que plus la profondeur de séquençage est élevée plus la probabilité de détecter le k-mer est élevée. Or, en raison des erreurs de séquençage, il est difficile d'affirmer que le k-mer est effectivement présent dans le génome ou bien que le k-mer détecté dans les reads est le produit d'un autre k-mer entaché d'erreur de séquençage. Dans une variante privilégiée, la détection dépend de la profondeur de séquençage réelle pour la souche bactérienne dans laquelle on cherche la présence de la séquence SEQ.

A cet effet, un test **812** est mis en œuvre pour savoir si l'échantillon est un échantillon métagénomique, ou plus généralement un échantillon contenant plusieurs espèces différentes (plusieurs espèces bactériennes, de l'ADN humain, ou autre), ou un échantillon préparé à partir d'un isolat de la souche bactérienne. Dans ce dernier cas, une seule souche étant présente, tous les reads appartiennent à ladite souche et la profondeur de séquençage, notée « cov », est calculée, en **814,** par exemple selon la relation :

$$cov = \frac{N_r}{N_g} \qquad (10)$$

relation dans laquelle $N_r$ est le nombre total de bases comprises dans les reads et $N_g$ est le nombre de bases dans un génome de référence de l'espèce bactérienne à laquelle appartient la souche bactérienne, préférentiellement ayant une taille moyenne ou proche de la moyenne des tailles de génomes observées pour ladite espèce (e.g. Ng = 4,4 millions de pair de bases (Mbp) pour *Mycobacterium tuberculosis*).

Un nombre de copies, notée $N_{cov}$, devant être détectées dans les reads pour affirmer qu'un k-mer est effectivement présent dans le génome, est alors calculé en **816** selon la relation suivante :

$$N_{cov} = \tau \times cov \qquad (11)$$

où $\tau$ est un paramètre prédéterminé, tenant compte préférentiellement du taux d'erreur de séquençage de la technologie de séquençage utilisée, avantageusement compris entre 5% et 15%, de préférence supérieur ou égal à 10%, par exemple 10%. Pour ce dernier pourcentage et une profondeur de 100, il faut donc détecter 10 copies à l'identique d'un k-mer pour déterminer qu'il est effectivement présent dans les reads. Un taux de 10% permet notamment de détecter avec précision la présence d'un k-mer dans des reads produits par une plateforme Gridion de la société Oxford Nanopore Technology Ltd à l'aide du kit de préparation de librairies R9.4 de la même société. Ce taux permet également un détection de précision dans les reads produits par une technologie de séquençage de type SBS, par exemple la plateforme MiSeq de la société Illumina Inc.

La détection de la présence ou de l'absence de chaque k-mer $km_i$ de l'ensemble *KM* dans les reads est ensuite mise en œuvre en **818**. En particulier, le k-mer $km_i$ est détecté s'il existe au moins $N_{cov}$ copies à l'identique dans les reads. Le procédé se poursuit alors par l'étape **804** décrite précédemment.

Si l'échantillon comprend plusieurs espèces (test **812**), le procédé consiste à déterminer la profondeur de séquençage pour l'espèce bactérienne considérée. Plus particulièrement, un « binning taxonomique » est mis en œuvre en **820,** un tel binning consistant à attribuer à chaque reads une origine parmi les espèces présentes dans l'échantillon. Ce type de binning est bien connu de l'état de la technique et met en œuvre par exemple la classification décrite dans l'article de Wood DE et al., « Kraken: ultrafast metagenomic sequence classification using exact alignments », Genome Biology, 2014, ou telle que mise en œuvre par le logiciel « Kraken2 » téléchargeable à l'adresse https://github.com/DerrickWood/kraken2/releases.

La profondeur de séquençage de ladite espèce bactérienne considérée est ensuite calculée, par exemple selon l'une ou l'autre des relations suivantes :

$$cov = \frac{N_r^m}{N_g} \qquad (12)$$

$$cov = \rho \times \frac{N_r}{N_g} \qquad (13)$$

relation dans laquelle $N_r^m$ est le nombre total de bases comprises dans les reads assignés à l'espèce bactérienne à laquelle appartient la souche bactérienne considérée, et $N_g$ est le nombre de bases dans un génome de taille moyenne de l'espèce bactérienne, et $\rho$ est la proportion relative de l'espèce bactérienne dans l'échantillon. Cette proportion relative est calculée par exemple au moyen la classification décrite dans l'article de Wood DE et al., « Kraken: ultrafast metagenomic sequence classification using exact alignments », Genome Biology, 2014, ou telle que mise en œuvre par le logiciel « Kraken2 » téléchargeable à l'adresse https://github.com/DerrickWood/kraken2/releases. Le procédé se poursuit par l'étape **816** de calcul du nombre de copie en fonction de la profondeur de séquençage venant d'être calculée.

En se référant de nouveau à l'étape **810** de détection de l'ensemble de séquences génomiques, noté {$SEQ_1$, ..., $SEQ_i$, ... $SEQ_E$}, cette étape fait suite à la détection de chaque séquence $SEQ_i$ de la manière décrite précédemment. Plus particulièrement, la détection dudit ensemble est mise en œuvre selon l'une des options suivantes :

i. l'ensemble est détecté comme présent dans le génome si le pourcentage des séquences $SEQ_i$ déterminées comme présentes est supérieur à un second seuil de détection prédéterminé $s_{clus}$, par exemple un seuil supérieur ou égal à 20%, par exemple 25%, et absent sinon ; ou

ii. l'ensemble est détecté comme présent dans le génome si toutes les séquences $SEQ_i$ sont déterminés comme présentes, et absent sinon ; ou

iii. l'ensemble est détecté comme présent dans le génome lorsqu'au moins une des séquences $SEQ_i$ est déterminée comme présente, et absent sinon ; ou

iv. l'ensemble est détecté comme présent dans le génome avec une probabilité égale au pourcentage de séquences $SEQ_i$ déterminées comme présentes.

Tout d'abord, on note que les performances de détection des séquences SEQ ou des ensembles de SEQ sont très similaires à celle obtenues directement par l'identification de k-mers, comme en atteste les performances d'un procédé à base de cluster-lasso comparativement aux performances d'un procédé à base de lasso telles que décrites précédemment.

Ensuite le procédé de détection **50** est robuste vis-à-vis du type de technologie de séquençage employée, et notamment de leur erreurs de séquençage. Le tableau suivant illustre, pour 37 souches de test de l'espèce *Klebsiella pneumoniae* sous forme d'isolat, avec une détection de variants (pourcentage 70%, 80%, 90%) pour les unitigs (équivalent des séquences $SEQ_i$) et une détection des motifs selon l'option i) ci-dessus, les résultats d'une prédiction cluster-lasso de la résistance à différents antibiotiques pour un séquençage par un MiSeq (« Illumina ») et par un Gridion (« ONT »). Les deux technologies de séquençage sont testées en fonction de leurs reads et des contigs produits par l'assemblage de leurs reads. A la lecture du tableau 10, on note que les résultats sont similaires pour les deux technologies, alors qu'elles présentent des taux d'erreurs de séquençage sensiblement différents, mais également des résultats similaires qu'il s'agisse de reads ou de contigs.

| | Type de donnée | bACC | Sensibilité | Spécificité | AUC |
|---|---|---|---|---|---|
| **Pipéracilline** | Reads Illumina | 86,5 | 81,5 | 100 | 91,1 |
| | Contigs Illumina | 86,5 | 81,5 | 100 | 92,2 |
| | Reads ONT | 89,2 | 85,2 | 100 | 91,9 |
| | Contigs ONT | 86,5 | 81,5 | 100 | 91,1 |
| **Céfépime** | Reads Illumina | 94,6 | 92,3 | 100 | 98,3 |
| | Contigs Illumina | 97,3 | 96,2 | 100 | 98,6 |
| | Reads ONT | 94,6 | 92,3 | 100 | 96,5 |
| | Contigs ONT | 97,3 | 96,2 | 100 | 98,3 |
| **Méropénème** | Reads Illumina | 86,5 | 81,0 | 93,3 | 92,7 |
| | Contigs Illumina | 86,5 | 81,0 | 93,8 | 92,7 |
| | Reads ONT | 78,4 | 61,9 | 100 | 86,9 |
| | Contigs ONT | 86,5 | 81,0 | 93,8 | 92,6 |
| **Ciprofloxacine** | Reads Illumina | 94,6 | 96,0 | 91,7 | 95,7 |
| | Contigs Illumina | 94,6 | 96,0 | 91,7 | 95,7 |
| | Reads ONT | 91,9 | 88,0 | 100 | 96,7 |
| | Contigs ONT | 94,6 | 96,0 | 91,7 | 95,7 |

Tableau 10

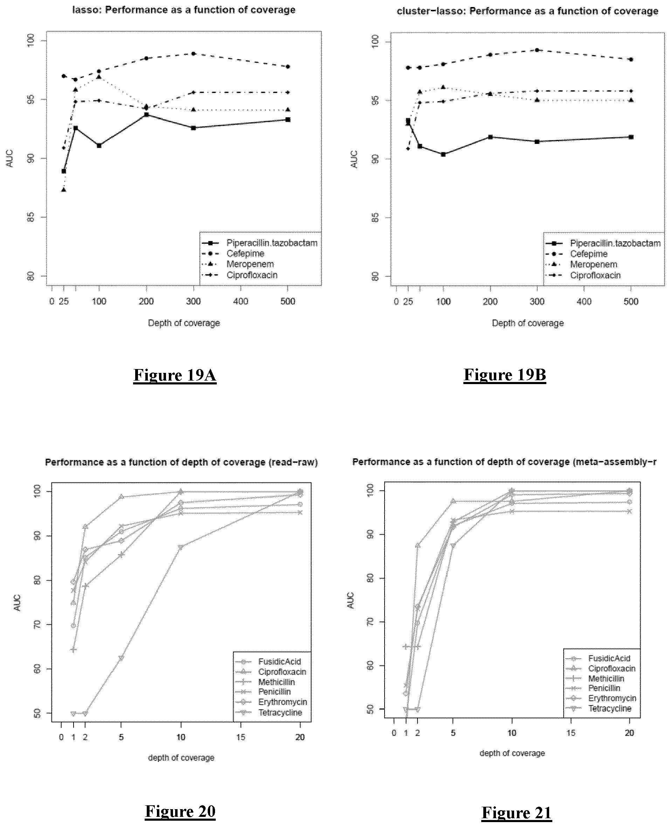

Par ailleurs, les performances de la prédiction (AUC) en fonction de la profondeur de séquençage pour la technologies ONT sont illustrées aux figures 19A (pour une prédiction lasso) et 19B (pour une prédiction cluster lasso), les échantillons étant produit à partir d'isolats des souches de *Klebsiella pneumoniae* . On note que la prise en compte de la profondeur de séquençage, et donc un nombre de copies croissant avec cette dernière, permet d'obtenir des performances rapidement stables, dès une profondeur de 30. Les figures 20 et 21 illustrent, au travers d'une étude de simulation, l'effet d'un échantillon métagénomique comprenant des souches de *Staphylococcus aureus* (en simulant ici des échantillons cliniques issus de lavage broncho-alvéolaire) sur les performances d'une prédiction cluster-lasso en fonction de reads (figures 20) ou de contigs (figure 21) pour la technologie Illumina. On note que également une stabilisation très rapide à de haute performance en fonction de la profondeur de séquençage réelle des souches *Staphylococcus aureus* présente dans l'échantillon.

D.2. Concernant d'autres caractéristiques du mode de réalisation

Il a été décrit un mode de réalisation particulier de l'invention. Ce procédé peut être modifié conformément aux caractéristiques suivantes, prises seules ou en combinaison :

- il a été décrit la prédiction à la susceptibilité à un antibiotique. L'invention s'applique à tout type de phénotype, par exemple la virulence d'une souche bactérienne, son ribotype, etc...
- il a été décrit l'application de l'invention à un échantillon biologique prélevé sur un patient. L'invention s'applique à tout type d'échantillon comprenant des bactéries, en particulier un échantillon prélevé sur un animal ou un échantillon prélevé dans l'environnement ;
- il a été décrit des bactéries. L'invention s'applique également aux levures et moisissures ;
- il a été décrit le séquençage complet du génome bactérien. En variante, le séquençage du génome est partiel et cible une ou plusieurs régions spécifiques connues pour leur implication de la susceptibilité à l'antibiotique ;
- dans le mode de réalisation décrit, la valeur des k-mers et des unitigs dans le génome est binaire (absence ou présence) telle que codée par exemple dans la matrice X. En variante, la valeur des k-mers ou des unitigs est égale au nombre de copies de ceux-ci dans le génome ;

- il a été décrit une prédiction binaire (états S et NS). En variante, la susceptibilité est ordinale (nombre d'états supérieur, par exemple S, R et I) ou linéaire (par exemple prédiction de la concentration minimale inhabité, ou « MIC »). Dans ce cas, la régression est ordinale ou linéaire.
- il a été décrit des modèles de prédiction appris par une régression logistique de type LASSO. D'autres algorithmes parcimonieux sont possibles, comme par exemple des modèles de forêts aléatoires (« random forest »), « gradient boosting », « set covering machine », par agrégation et Monte-Carlo ou par apprentissage profond, ou par tout type d'apprentissage pénalisé de type Lasso (elastic-net, group-lasso, fused lasso, adaptative lasso, ...)
- il a été décrit une sélection des motifs MAF à l'aide d'un régression logistique lasso. D'autres sélections sont possibles, par exemple une telle que décrite dans l'article Friedman J.H. « Greedy Function Approximation: A Gradient Boosting Machine de sélection », The Annals of Statistics, 2001, et telle que mise en œuvre par exemple par le logiciel « xGBoost », accessible à l'adresse https://xgboost.readthedocs.io/en/latest/, ou tout autre sélection non-linéaire.
- il a été décrit un clustering basé sur des valeurs de la corrélation de Bravais-Pearson. D'autres type de mesure de la co-occurrence sont possibles, par exemple une distance de Jaccard ou de Sørensen-Dice.
- il a été décrit un clustering particulier. D'autres types de clustering sont possibles, par exemple un clustering hiérarchique dit « standard ».
- la valeur d'un cluster a été décrite comme étant égale à la moyenne des motifs le constituant. D'autres valeurs sont possibles. Par exemple, une régression logistique de type « groupe lasso » est mise en œuvre pour chacun des clusters afin d'attribuer des poids différents aux différents motifs le constituant.
- il a été décrit la mise en œuvre d'algorithmes d'apprentissage à partir degénomes assemblés. En variante, l'invention s'applique directement aux génomes produit par une plateforme de séquençage, i.e à des génomes sous forme de « reads », optionnellement filtré des reads de faible qualité.

## Revendications

1. Procédé de détermination d'une caractéristique phénotypique d'une souche bactérienne, comprenant :

   - le séquençage (400) partiel ou total du génome de ladite souche;
   - l'application (402, 404), au génome séquencé, d'un modèle prédéterminé de prédiction de ladite caractéristique phénotypique, ledit modèle ayant pour variables des groupes de séquences génomiques,

   *caractérisé* **en ce que** lesdits groupes sont sélectionnés (304, 312) de sorte :

   - qu'un taux de co-occurrence, dans le génome de l'espèce bactérienne, des séquences génomiques constitutives desdits groupes est supérieur à un seuil prédéterminé (304); et
   - que les groupes sont des regroupements desdites séquences génomiques en fonction de leurs taux de co-occurrence dans le génome de l'espèce bactérienne (312).

2. Procédé selon la revendication 1, dans lequel un premier sous ensemble des séquences génomiques est un ensemble de variables non groupées d'un premier modèle de prédiction de la caractéristique phénotypique (314).

3. Procédé selon la revendication 2, dans lequel un second sous ensemble des séquences génomiques est un ensemble de séquences génomiques ayant un taux de co-occurrence avec le premier ensemble supérieur au seuil prédéterminé (316).

4. Procédé selon la revendication 2 ou 3, en ce que le premier modèle de prédiction est un modèle appris sur une base de données de génomes et de caractéristique phénotypique de souches bactériennes appartenant à l'espèce bactérienne, l'apprentissage appartenant à la classe des apprentissages parcimonieux, en particulier à base de régression logistique.

5. Procédé selon la revendication 4, dans lequel l'apprentissage est réalisé par une régression logistique de type LASSO.

6. Procédé selon l'une des revendications précédentes, dans lequel les séquences génomiques correspondent à des nœuds d'un graphe compacté obtenu à partir de séquences génomiques de longueur constante et occurrentes dans des génomes de souches appartenant à l'espèce bactérienne (306, 308).

**7.** Procédé selon la revendication 6, dans lequel ledit graphe compacté est calculé en

- calculant un graphe de De Buijn d'alphabet A, T, G, C à partir desdites séquences génomiques de longueur constante ;
- en compactant des chemins linéaires du graphes de De Bruijn de manière à obtenir ledit graphe compacté.

**8.** Procédé selon l'une quelconque des revendications précédentes, dans lequel le regroupement des séquences génomiques comporte :

- l'obtention de premiers groupes de séquences génomiques par l'application d'un regroupement des séquences ayant une co-occurence parfaite dans des génomes de souches appartenant à l'espèces bactérienne (310);
- l'obtention des groupes du modèle de prédiction par l'application d'un second regroupement desdits premiers groupes basé sur les taux de co-occurrence desdits premiers groupes dans lesdits génomes (312).

**9.** Procédé selon l'une quelconque des revendications précédentes, dans lequel le regroupement des séquences génomiques (312) comporte (318):

- le calcul d'un dendrogramme en fonction des taux de co-occurrence des séquences génomiques dans des génomes de souches appartenant à l'espèce bactérienne; et
- la partition du dendrogramme à une hauteur prédéterminée de manière à obtenir les groupes de séquences génomiques.

**10.** Procédé selon les revendications 8 et 9, dans lequel le dendrogramme est calculé sur les premiers groupes de séquences génomiques.

**11.** Procédé selon l'une quelconque des revendications précédentes, dans lequel le modèle de prédiction est appris sur une base de données de génomes et de caractéristiques phénotypiques de souches bactériennes appartenant à l'espèce bactérienne, l'apprentissage appartenant à la classe des apprentissages parcimonieux, en particulier à base de régression logistique (322).

**12.** Procédé selon la revendication 11, dans lequel l'apprentissage est réalisé par une régression logistique de type LASSO.

**13.** Procédé selon la revendication 11 ou 12, dans lequel la valeur d'un groupe de séquences génomiques pour l'apprentissage parcimonieux est calculé (328) :

- pour chaque séquence génomique appartenant audit groupe, en calculant un vecteur d'occurrences de ladite séquence dans les génomes de la base de données ;
- en calculant le vecteur moyen des vecteurs d'occurrences, ledit vecteur moyen constituant la valeur dudit groupe.

**14.** Procédé selon l'une quelconque des revendications précédentes, dans lequel la valeur d'un groupe de séquences génomiques pour l'application du modèle de prédiction appliqué à la souche bactérienne est égale au pourcentage de séquences génomiques dudit groupe présentes dans le génome de la souche bactérienne.

**15.** Procédé selon la revendication 8, dans lequel la valeur d'un groupe de séquences génomiques pour l'application du modèle de prédiction appliqué à la souche bactérienne est égale au pourcentage desdites premiers groupes dudit groupe présents dans le génome de la souche bactérienne (328).

**16.** Procédé selon la revendication 8, dans lequel la valeur d'un groupe de séquences génomiques pour l'application du modèle de prédiction appliqué à la souche bactérienne est calculée (328) en :

- calculant, pour chaque premier groupe dudit groupe, le pourcentage de séquences génomiques dudit premier groupe présentes dans le génomes de la souche bactérienne ; et
- en calculant la valeur comme étant égale à la moyenne des pourcentages des premiers groupes dudit groupe.

**17.** Procédé selon la revendication précédente, dans lequel une séquence génomique est présente dans le génome de la souche bactérienne lorsqu'un pourcentage de séquences génomiques de longueur constante constitutives de ladite séquence est supérieur à un seuil prédéterminé dépendant de la longueur de ladite séquence.

18. Procédé d'identification de signatures génomiques prédictives d'une caractéristique phénotypique d'une espèce bactérienne, le procédé comprenant :

- la constitution (300) d'une base de données de génomes et de caractéristique phénotypique d'une pluralité de souches bactériennes appartenant à l'espèce bactérienne ;
- le calcul (306, 308, 310) d'un ensemble de séquences génomiques descriptives des génomes de la base de données, et pour chacune desdites séquences le calcul d'un vecteur d'occurrence de ladite séquence dans les génomes de la base de données ;
- la sélection (314, 316), dans ledit ensemble de séquences génomiques, des séquences ayant un taux de co-occurrence supérieur à un seuil prédéterminé, ledit taux étant calculé en fonction des vecteurs d'occurrence ;
- le regroupement (318) des séquences génomiques sélectionnées en fonction de leurs taux de co-occurrence, de manière à obtenir des groupes de séquences génomiques ;
- l'apprentissage (326) d'un modèle de prédiction de la caractéristique phénotypique de l'espèce bactérienne à l'antibiotique ayant pour variables les groupes de séquences génomiques ;
- l'identification (328) de la signature comme étant égale à l'union de groupes de séquences génomiques.

19. Procédé selon la revendication 18, dans lequel la sélection des séquences génomiques comprend :

- la sélection d'un premier ensemble des séquences génomiques constitué de variables non groupées d'un premier modèle de prédiction de la caractéristique phénotypique;
- la sélection, parmi les séquences génomiques non sélectionnées lors de la première sélection, de séquences génomiques ayant un taux de co-occurrence avec les séquences génomiques du premier ensemble supérieur au seuil prédéterminé.

20. Procédé selon la revendication 18, en ce que le premier modèle de prédiction est un modèle appris sur une base de données de génomes et de caractéristiques phénotypiques de souches bactériennes appartenant à l'espèce bactérienne, l'apprentissage appartenant à la classe des apprentissages parcimonieux.

21. Procédé selon la revendication 20, dans lequel l'apprentissage est réalisé par une régression logistique, notamment de type LASSO.

22. Procédé selon l'une des revendications 18 à 21, dans lequel les séquences génomiques correspondent nœuds d'un graphe compacté obtenu à partir de séquences génomiques de longueur constante et occurrentes dans des génomes de souche appartenant à l'espèce bactérienne.

23. Procédé selon la revendication 22, dans lequel ledit graphe compacté est calculé en

- calculant un graphe de De Buijn d'alphabet A, T, G, C à partir desdites séquences génomiques de longueur constante ;
- en compactant des chemins linéaires du graphes de De Bruijn de manière à obtenir ledit graphe compacté.

24. Procédé selon l'une quelconque des revendications 18 à 23, dans lequel le regroupement des séquences génomiques sélectionnées comporte :

- l'obtention de premiers groupes de séquences génomiques par l'application d'un regroupement des séquences ayant une co-occurente parfaite dans des génomes de souches appartenant à l'espèces bactérienne ;
- l'obtention des groupes du modèle de prédiction par l'application d'un second regroupement en fonction des taux de co-occurrence des premiers groupes de séquences dans lesdits génomes.

25. Procédé selon l'une quelconque des revendications 18 à 24, dans lequel le regroupement des séquences génomiques sélectionnées comporte :

- le calcul d'un dendrogramme en fonction des taux de co-occurrence des séquences génomiques dans des génomes de souches appartenant à l'espèce bactérienne ; et
- la partition du dendrogramme à une hauteur prédéterminée de manière à obtenir les groupes de séquences génomiques.

**26.** Procédé selon les revendications 24 et 25, dans lequel le dendrogramme est calculé sur les premiers groupes de séquences génomiques.

**27.** Procédé selon l'une quelconque des revendications 18 à 26, dans lequel le modèle de prédiction est appris sur une base de données de génomes et de caractéristiques phénotypiques de souches bactériennes appartenant à l'espèce bactérienne, l'apprentissage appartenant à la classe apprentissages parcimonieux.

**28.** Procédé selon la revendication 27, dans lequel l'apprentissage est réalisé par une régression logistique, notamment de type LASSO.

**29.** Procédé selon la revendication 27 ou 26, dans lequel la valeur d'un groupe de séquences génomiques pour la régression logistique est calculé en

- pour chaque séquence génomique appartenant audit groupe, en calculant un vecteur d'occurrence de ladite séquence dans les génomes de la base de données ;
- en calculant le vecteur moyen des vecteurs d'occurrence, ledit vecteur moyen constituant la valeur dudit groupe.

**30.** Procédé selon l'une quelconque des revendications précédentes, dans lequel la caractéristique phénotypique est la susceptibilité à un antibiotique de la souche bactérienne.

**31.** Utilisation d'une signature génomique identifiée selon un procédé conforme à l'une des revendications 18 à 29, dans un test moléculaire de prédiction de la caractéristique phénotypique, en particulier un test de type réaction en chaîne par polymérase, ou un test à base de puce à ADN, ledit test ciblant au moins partiellement la signature génomique.

**32.** Produit programme d'ordinateur mémorisant des instructions exécutables par un ordinateur pour l'application d'un modèle de prédiction d'unecaractéristique phénotypique, en particulier de la susceptibilité à un antibiotique, d'une souche bactérienne, ladite application étant conforme à l'une quelconque des revendications 1 à 17.

**33.** Système de détermination d'une caractéristique phénotypique, en particulier de la susceptibilité à un antibiotique, d'une souche bactérienne, comprenant :

- un plateforme de séquençage pour le séquençage (400) partiel ou total du génome de ladite souche;
- une unité informatique configurée pour l'application (402, 404), au génome séquencé, d'un modèle prédéterminé de prédiction de la caractéristique phénotypique, conforme à l'une quelconque des revendications 1 à 17.

**34.** Produit programme d'ordinateur mémorisant des instructions exécutables par un ordinateur pour la mise en œuvre d'un procédé d'identification de signatures génomiques prédictives d'une caractéristique phénotypique, en particulier de la susceptibilité à un antibiotique d'une souche bactérienne, le procédé étant conforme à l'une quelconque des revendications 18 à 29.

**Figure 1**

30

```
┌─────────┐
│   300   │
└─────────┘
     │
     ▼
┌─────────┐
│   302   │
└─────────┘
     │
┌ ─ ─ ┼ ─ ─ ─ ─ ─ ┐          304
     │
│    ▼          │
┌─────────┐
││   306   │     │
└─────────┘
│    │          │
     ▼
│┌─────────┐    │
 │   308   │
│└─────────┘    │
     │
│    ▼          │
┌─────────┐
││   310   │     │
└─────────┘
└ ─ ─ ┼ ─ ─ ─ ─ ─ ┘
┌ ─ ─ ┼ ─ ─ ─ ─ ─ ┐          312
     ▼
│┌─────────┐    │
 │   314   │
│└─────────┘    │
     │
│    ▼          │
┌─────────┐
││   316   │     │
└─────────┘
│    │          │
     ▼
│┌─────────┐    │
 │   318   │
│└─────────┘    │
└ ─ ─ ┼ ─ ─ ─ ─ ─ ┘
┌ ─ ─ ┼ ─ ─ ─ ─ ─ ┐          322
     ▼
│┌─────────┐    │
 │   324   │
│└─────────┘    │
     │
│    ▼          │
┌─────────┐
││   326   │     │
└─────────┘
│    │          │
┌ ─ ┼ ─ ─ ┐     328
│    ▼    │    │
│┌─────────┐   │
  │   330   │   │
││└─────────┘   │
     │    │
│    ▼    │    │
│┌─────────┐   │
  │   332   │   │
││└─────────┘   │
│ ─ ─ ─ ─ ─ ┘   │
└ ─ ─ ─ ─ ─ ─ ─ ┘
```

**Figure 2A**

40

```
┌─────────┐
│   12    │
└─────────┘
     │
┌ ─ ─ ┼ ─ ─ ─ ─ ─ ┐
     ▼
│┌─────────┐    │
 │   400   │
│└─────────┘    │
     │
│    ▼          │
┌─────────┐
││   402   │     │
└─────────┘
│    │          │
     ▼
│┌─────────┐    │
 │   406   │
│└─────────┘    │
└ ─ ─ ┼ ─ ─ ─ ─ ─ ┘
     │
     ▼
┌─────────┐
│   14    │
└─────────┘
```

**Figure 2B**

**Figure 4**

**Figure 5**

**Figure 3**

**A. Graph compaction: from k-mers to unitigs**

Input genomes (contigs) are k-merized to build a de Bruijn graph (DBG)

G

| Strains | | |
|---|---|---|
| | 1 | TTCG▢TCGTA |
| | 2 | TTCGATCGTA |
| | 3 | TTCG▢TCGTA |
| | 4 | TTCGATCGTA |
| | 5 | TTCGATCGTA |

K-merization +DBG

**1. DBG**
(not compacted: each node is a k-mer)

DBG

TTCG → TCGC → CGCT → GCTC → CTCG → TCGT → CGTA
TTCG → TCGA → CGAT → GATC → ATCG → TCGT

Compaction

**2. cDBG**
(compacted: each node is a unitig representing 1 or more equivalent k-mers)

cDBG

TTCG → TCGC TCG → TCGTA
TTCG → TCGATCG → TCGTA

**B. Matrix deduplication: from unitigs to features**

**1.** Each unitigs of the cDBG is encoded by a variable representing its presence in each strain:

| Features: unitig | | | | |
|---|---|---|---|---|
| Strains | TTCG | TCGTA | TCGATCG | TCG▢TCG |
| 1 | 1 | 1 | 0 | 1 |
| 2 | 1 | 1 | 1 | 0 |
| 3 | 1 | 1 | 0 | 1 |
| 4 | 1 | 1 | 1 | 0 |
| 5 | 1 | 1 | 1 | 0 |

V

Deduplication +MAF

**2.** Unitigs presences are converted in minor allele frequency (MAF) patterns. Identical patterns are merged.

| Features: MAF patterns | | |
|---|---|---|
| Strains | TTCG TCGTA | TCG▢TCG TCGATCG |
| 1 | 0 | 1 |
| 2 | 0 | 0 |
| 3 | 0 | 1 |
| 4 | 0 | 0 |
| 5 | 0 | 0 |

W

**3.** Patterns with a low MAF are removed

| MAF > 1% | |
|---|---|
| Strains | TCG▢TCG TCGATCG |
| 1 | 1 |
| 2 | 0 |
| 3 | 1 |
| 4 | 0 |
| 5 | 0 |

X

**Figure 6**

Figure 7

LASSO MODEL

CLUSTER-LASSO MODEL

tetracycline : absolute values of model coefficients

tetracycline : absolute values of model coefficients

CLUSTER-LASSO MODEL

Nombre d'unitigs poour les 10 clusters les plus larges

**Figure 8**

LASSO MODEL

CLUSTER-LASSO MODEL

TetB

TetA/TetR

First subgraph

First subgraph

TetB

TetB/TetD/TetR

Second subgraph

Second subgraph

**Figure 9**

LASSO MODEL

CLUSTER-LASSO MODEL

gentamicin : absolute values of model coefficients

gentamicin : absolute values of model coefficients

CLUSTER-LASSO MODEL

Nombre d'unitigs pour les 10 clusters les plus larges

**Figure 10**

LASSO MODEL

CLUSTER-LASSO MODEL

AAC3

AAC3, (Bla)OXA, (Bla)IMP, (Bla)TEM, (Bla)CARB, (Phe)CATB

First subgraph

No annotation

First subgraph:
cluster #{1, 3, 4, 5, 6, 7, 8, 10, 13, 14, 18, 21, 27, 29, 33}

Second subgraph

AAC3/TEM

Third subgraph

**Figure 11**

Figure 12

LASSO MODEL

*penA*

First subgraph

*penA*

Second subgraph

CLUSTER-LASSO MODEL

*penA*

First subgraph

**Figure 13**

LASSO MODEL

CLUSTER-LASSO MODEL

Tetracycline : absolute values of model coefficients

Tetracycline : absolute values of model coefficients

CLUSTER-LASSO MODEL

Nombre d'unitigs pour les 10 clusters les plus larges

**Figure 14**

**Figure 15**

**Figure 16**

**Figure 18**

**Figure 17**

lasso: Performance as a function of coverage

**Figure 19A**

cluster-lasso: Performance as a function of coverage

**Figure 19B**

Performance as a function of depth of coverage (read-raw)

**Figure 20**

Performance as a function of depth of coverage (meta-assembly-r

**Figure 21**

Europäisches
Patentamt

European
Patent Office

Office européen
des brevets

## RAPPORT DE RECHERCHE EUROPEENNE

Numéro de la demande

EP 20 16 2655

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | PIERRE MAHÉ ET AL: "Predicting bacterial resistance from whole-genome sequences using -mers and stability selection", BMC BIOINFORMATICS, BIOMED CENTRAL LTD, LONDON, UK, vol. 19, no. 1, 17 octobre 2018 (2018-10-17), pages 1-11, XP021261641, DOI: 10.1186/S12859-018-2403-Z * Résumé ; page 2/11-4/11 , "Methods" * ----- | 1-34 | INV. G16B30/00 G16B20/00 G16B40/20 |
| X | MAGALI JAILLARD ET AL: "A fast and agnostic method for bacterial genome-wide association studies: Bridging the gap between k-mers and genetic events", PLOS GENETICS, vol. 14, no. 11, 12 novembre 2018 (2018-11-12), page e1007758, XP055738526, DOI: 10.1371/journal.pgen.1007758 * page 13/28-15/28 , "Methods"; figure 1 * ----- | 1,6-8, 18,31-34 | |

DOMAINES TECHNIQUES RECHERCHES (IPC)

G16B

Le présent rapport a été établi pour toutes les revendications

| Lieu de la recherche | Date d'achèvement de la recherche | Examinateur |
|---|---|---|
| Munich | 13 octobre 2020 | Schmidt, Karsten |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
.............................................................
& : membre de la même famille, document correspondant

EPO FORM 1503 03.82 (P04C02)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **DE M. JAILLARD DANCETTE.** *Vers une cartographie des polymormismes liés à la résistance aux antimicrobiens,* 2018
- **M. JAILLARD DANCETTE.** *Vers une cartographie des polymorphismes liés à la résistance aux antimicrobiens,* 2018
- **DE JAILLARD M. et al.** A fast and agnostic method for genome-wide association studies : Bridging the gap between k-mers et genetic events. *PLOS Genetics,* 2018
- **DE FRIEDMAN et al.** Regularization Paths for Generalized Linear Models via Coordinate Descent. *Journal of Statistical Software,* 2010
- **DE BÜHLMANN P. et al.** Correlated variables in regression : Clustering and sparse estimation. *Journal of Statisical Planning and Inference,* 2013
- **NOVAIS A. et al.** Spread of an OmpK36-modified ST15 Klebsiella pneumoniae variant during an outbreak involving multiple carbapenem-resistant Enterobacteriaceae species and clones. *European Journal of clinical microbiology and infectious deseases,* 2012
- **DE WOOD DE et al.** Kraken: ultrafast metagenomic sequence classification using exact alignments. *Genome Biology,* 2014
- **FRIEDMAN J.H.** Greedy Function Approximation: A Gradient Boosting Machine de sélection. *The Annals of Statistics,* 2001